# EUROPEAN PATENT SPECIFICATION

(11) **EP 3 432 872 B1**
(45) Date of publication and mention of the grant of the patent: **25.08.2021**
(21) Application number: 17770846.8
(22) Date of filing: 16.03.2017
(51) Int. Cl.: A61K 31/195, A61K 31/197, A61P 35/04

(54) **EFLORNITHINE FOR USE IN THE TREATMENT OF TEMOZOLOMIDE RECURRENT/REFRACTORY ANAPLASTIC ASTROCYTOMA**
EFLORNITHIN ZUR VERWENDUNG IN DER BEHANDLUNG VON TEMOZOLOMID RESISTENTEM/WIEDERKEHRENDEM ANAPLASTISCHEM ASTROZYTOM
ÉFLORNITHINE POUR L'UTILISATION DANS LE TRAITEMENT D'ASTROCYTOME ANAPLASTIQUE RÉCURRENT/RÉSISTANT AU TRAITEMENT AVEC LE TEMOZOLOMIDE

(30) Priority: 24.03.2016 US 201662312623 P
(43) Date of publication of application: 30.01.2019
(73) Proprietor: Orbus Therapeutics, Inc., Palo Alto, CA 94303 (US)
(72) Inventor: LEVIN, Victor, A., Palo Alto, CA 94303 (US)
(74) Representative: Carpmaels & Ransford LLP
(86) International application number: PCT/US2017/022718
(87) International publication number: WO 2017/165187

(56) References cited:
- EP-B1- 0 046 713
- US-A1- 2003 040 526
- US-A1- 2010 120 727
- US-A1- 2015 297 553
- US-A1- 2015 306 241
- US-B1- 6 949 679
- LEVIN VICTOR A ET AL: "Phase III Randomized Study of Postradiotherapy Chemotherapy with alpha-Difluoromethylornithine-Procarbazine , N-(2-Chloroethyl)-N' -cyclohexyl-N-nitrosurea, Vincristine (DFMO-PCV) Versus PCV for Glioblastoma Multiforme", CLINICAL CANCER RESEARCH : AN OFFICIAL JOURNAL OF THE AMERICAN ASSOCIATION FOR CANCER RESEARCH, vol. 6, no. 10, October 2000 (2000-10), pages 3878-3884, XP002794644, ISSN: 1078-0432
- LEVIN V A ET AL: "PHASE I-II STUDY OF EFLORNITHINE AND MITOGUAZONE COMBINED IN THE TREATMENT OF RECURRENT PRIMARY BRAIN TUMORS", CANCER TREATMENT REPORTS, vol. 71, no. 5, 1987, pages 459-464, XP009516281, ISSN: 0361-5960
- LEVIN V A ET AL: "TREATMENT OF RECURRENT GLIOMAS WITH EFLORNITHINE", JOURNAL OF THE NATIONAL CANCER INSTITUTE (BETHESDA), vol. 84, no. 18, 1992, pages 1432-1437, XP009516286, ISSN: 0027-8874
- REGENASS, U ET AL.: 'CGP 48664, a New S-Adenosylmethionine Decarboxylase Inhibitor with Broad Spectrum Antiproliferative and Antitumor Activity' CANCER RESEARCH vol. 54, 15 June 1994, pages 13210 - 3217, XP001246981

## Description

This application claims the benefit of United States Provisional Patent Application Serial No. 62/312,623 by Victor A. Levin, M.D., filed March 24, 2016 and entitled "Compositions and Methods for Use of Eflornithine and Derivatives and Analogs Thereof to Treat Cancers, Including Gliomas".

### FIELD OF THE INVENTION

This invention is defined in the appended claims. It is directed to compositions and methods for the use of eflornithine or a pharmaceutically acceptable salt thereof to treat temozolomide recurrent/refractory anaplastic astrocytoma.

### BACKGROUND OF THE INVENTION

Glioma is one of the most common and serious form of brain tumor. Gliomas are classified by cell type, by grade, and by location. Gliomas are generally named according to the specific type of cell with which they share histological features. These are not necessarily the cell types from which the glioma originated. The main types of glioma are: ependyoma (ependymal cells), astrocytoma (astrocytes), oligodendroglioma (oligodendrocytes), brainstem glioma (brain stem), optic nerve glioma (cells in or around the optic nerve), and mixed glioma (cells from different types of glia). Gliomas are further characterized according to their grade, generally stated according to the WHO classification. Grade I is the lowest grade with the least advanced disease and the best prognosis, and Grade I gliomas are generally considered benign. Grade II of the WHO classification is the next lowest grade. Gliomas of Grade II are well-differentiated and not anaplastic. Although these tend to exhibit benign tendencies and can be associated with a favorable prognosis, they have a tendency to recur and to increase in grade, and thus, in severity, over time. High-grade gliomas, Grades III and IV in the WHO classification, are undifferentiated or anaplastic and are clearly malignant. These grades carry the worst prognosis. Gliomas can also be classified according to their location, specifically whether they are above or below a membrane in the brain, the tentorium. The tentorium separates the cerebrum from the cerebellum. Supratentorial gliomas are more common in adults, while infratentorial gliomas are more common in children. Certain types of glioma, such as subependymoma or juvenile pyelocytic astrocytoma (JPA) tend to be non-invasive or much less invasive.

The symptoms of glioma generally depend on which part of the central nervous system is affected. Gliomas in the brain can cause headaches, vomiting, seizures, focal weakness, problems forming new memories, problems with speech, and cranial nerve disorders as a result of tumor growth. Gliomas of the optic nerve can cause visual disturbances or vision loss. Gliomas of the spinal cord can cause pain, weakness, or numbness in one or more extremities. Generally, gliomas do not metastasize through the bloodstream, but can spread through the cerebrospinal fluid and cause drop metastases in the spinal cord.

The exact causes of gliomas are not known. Certain hereditary genetic disorders such as type 1 or type 2 neurofibromatosis or tuberous sclerosis can predispose to their development. A number of oncogenes can be involved in glioma initiation and development. Many gliomas are infected with cytomegalovirus, which can accelerate their development. Germ-line (inherited) polymorphisms of the DNA repair genes *ERCC1, ERCC2* (*XPD*) and *XRCC1* can increase the risk of glioma. This indicates that altered or deficient repair of DNA damage can contribute to the formation of gliomas. Excess DNA damage can give rise to mutations through translesion synthesis. Furthermore, incomplete DNA repair can give rise to epigenetic alterations or epimutations. Such mutations and epimutations may provide a cell with a proliferative advantage which can then, by a process of natural selection, lead to progression to cancer. Epigenetic repression of DNA repair genes is often found in progression to sporadic glioblastoma. For instance, methylation of the DNA repair gene *MGMT* promoter was observed in a substantial fraction of glioblastomas. In addition, in some glioblastomas, the MGMT protein is deficient due to another type of epigenetic alteration. MGMT protein expression may also be reduced due to increased levels of a microRNA that inhibits the ability of the *MGMT* messenger RNA to produce the MGMT protein. It was found that, in glioblastomas without methylated *MGMT* promoters, that the level of microRNA miR-181d is inversely correlated with protein expression of MGMT and that the direct target of miR-181d is the *MGMT* mRNA 3'UTR. Epigenetic reductions in expression of another DNA repair protein, *ERCC1,* were found in many gliomas; in some cases, the reduction was due to reduced or absent ERCC1 protein expression was reduced or absent. In other cases, the reduction was due to methylation of the *ERCC1* promoter. In a small number of cases, the reduction could have been due to epigenetic alterations in microRNAs that affect *ERCC1* expression. When expression of DNA repair genes is reduced, DNA damage can accumulate in cells at increased levels. In gliomas, mutations frequently occur in the isocitrate dehydrogenase genes *IDH1* and *IDH2.* These mutations may result in production of an excess metabolic intermediate, 2-hydroxyglutarate, which binds to catalytic sites in key enzymes that are important in altering histone and DNA promoter methylation. This may result in a DNA CpG island methylator phenotype (CIMP) that can cause promoter hypermethylation and concomitant silencing of tumor suppressor genes such as DNA repair genes *MGMT* and *ERCC1.* Additionally, mutations in *IDH1* and *IDH2* may cause increased oxidative stress and thus initiate increased oxidative damage to DNA.

Several acquired genetic mutations are commonly found in gliomas, including mutations in p53 and PTEN; the gene encoding PTEN may also be lost. These mutations can lead to overexpression of *EGFR.* However, hypermutation associated with gliomas is not confined to specific locations.

High-grade gliomas are highly vascular tumors and have a tendency to infiltrate. They have extensive areas of necrosis and hypoxia. Often, tumor growth causes a breakdown of the blood-brain barrier in the vicinity of the tumor. As a rule, high-grade gliomas almost always grow back even after complete surgical excision, so are commonly called recurrent cancer of the brain. In contrast, lower-grade gliomas typically grow relatively slowly and can be followed without the need for aggressive treatment unless they grow or cause symptoms.

Treatment for gliomas depends on the location, the cell type, and the grade of malignancy. A combined approach, including surgical resection, radiotherapy, and chemotherapy, is frequently employed. One therapeutic agent frequently employed is temozolomide, which can cross the blood-brain barrier and is frequently used in treatment of higher-grade gliomas. The angiogenic blocker bevacizumab, a monoclonal antibody, is also frequently used. However, there is increasing evidence that the use of temozolomide may itself induce mutations and worsen prognosis in a significant fraction of patients (B.E. Johnson et al., "Mutational Analysis Reveals the Origin and Therapy-Driven Evolution of Recurrent Glioma," Science 343: 189-193 (2014), incorporated herein by this reference). The potentially mutagenic effect of temozolomide must be taken into account in planning a course of treatment for glioma.

Gliomas are rarely curable. The prognosis for patients with high-grade gliomas is generally poor, and is especially so for older patients. Of 10,000 Americans diagnosed each year with malignant gliomas and based on CBTRUS (table 23, 2015 edition), about 57% are alive one year after diagnosis, 41% after two years, and only 31% at five years. Those with anaplastic astrocytoma have about 44% at two years and 28% at five years. Glioblastoma multiforme has a worse prognosis with a 37% one year survival and 15% two year survival after diagnosis. For low-grade gliomas, the prognosis is somewhat more optimistic, but even such patients have a far higher death rate than does the general population when age is taken into account.

Therefore, there is a substantial need for an improved treatment for gliomas. In addition, there is a particular need to provide treatments that can avoid or counteract the potentially mutagenic effect of the frequently-used antineoplastic drugs,such as temozolomide. As detailed below, the principles of treatment provided in the present invention can also be applied to malignancies in general, as cancer is typically characterized by mutation of the neoplastic cells.

### SUMMARY OF THE INVENTION

The present invention provides a new therapeutic modality for the treatment of glioma.

One aspect of the present invention is a method for the treatment of glioma comprising the step of administering a therapeutically effective quantity of eflornithine or a pharmaceutically acceptable salt thereof to a subject with glioma in order to reduce the rate of mutation of the glioma to reduce the progression of the glioma, wherein the glioma is temozolomide recurrent/refractory anaplastic astrocytoma. The use of eflornithine is disclosed in United States Patent No. 6,553,351 by Levin.

In one alternative, the eflornithine or pharmaceutically acceptable salt thereof is a racemic mixture of D-eflornithine and L-eflornithine, D-eflornithine, or L-eflornithine. In another alternative, the eflornithine or pharmaceutically acceptable salt thereof is a pharmaceutically acceptable salt of eflornithine.

Typically, the eflornithine or pharmaceutically acceptable salt thereof reduces the rate of mutation of the glioma associated with the administration of an alkylating agent. The alkylating agent according to the invention is temozolomide. All alkylating agents are mutagenic to some degree.

The eflornithine or pharmaceutically acceptable salt thereof can be administered orally or by injection.

In one alternative, the glioma was previously treated with radiation therapy and adjuvant alkylator therapy and is temozolomide recurrent/refractory anaplastic glioma.

The use of radiation therapy is conventional for glioma (M.D. Prados et al., "Phase III Trial of Accelerated Hyperfractionation with or without Difluromethylornithine (DFMO) Versus Standard Fractionated Radiotherapy with or without DFMO for Newly Diagnosed Patients with Glioblastoma Multiforme," Int. J. Rad. Oncol. Biol. Phys. 49: 71-77 (2001)).

The glioma can have a mutation in one or more genes selected from the group consisting of *IDH1, IDH2, TP53, PTEN,* and *ATRX.* The glioma can have the promoter for *MGMT* methylated. According to the invention the form of glioma to be treated is astrocytoma.

The eflornithine or pharmaceutically acceptable salt thereof can be administered together with a therapeutically effective quantity of one or more conventional anti-neoplastic agents used for the treatment of glioma. The one or more conventional anti-neoplastic agents used for the treatment of glioma can be selected from the group consisting of alkylating agents, antimetabolites, anti-angiogenic agents, EGFR inhibitors, platinum-containing agents, topoisomerase inhibitors, and other classes of agents. In another alternative, the eflornithine or pharmaceutically acceptable salt thereof is administered together with an inhibitor of polyamine transport. In yet another alternative, the eflornithine or pharmaceutically acceptable salt thereof is administered together with a polyamine analog. In still another alternative, the eflornithine or pharmaceutically acceptable salt thereof is administered together with an S-adenosylmethionine decarboxylase inhibitor. In yet another alternative, the eflornithine or pharmaceutically acceptable salt thereof is administered together with an agent selected from the group consisting of: (1) a retinoid; (2) a syrbactin compound; (3) a cyclooxygenase-2 inhibitor; (4) a verinoid; (5) a non-steroidal anti-inflammatory agent; (5) castanospermine or castanospermine esters; (6) an aziridinyl putrescine compound; (7) an interferon; (8) an aryl substituted xylopyranoside derivative; (9) an agent that reduces blood glutamate levels and enhances brain to blood glutamate efflux; (10) chitosan or chitosan derivatives and analogs; (11) 2,4-disulfonyl phenyl tert-butyl nitrone; (12) 3-(4-amino-1-oxo-1,3-dihydro-isoindol-2-yl)-piperidine-2,6-dione; (13) thalidomide; (14) N-2-pyridinyl-2-pyridinecarbothioamide; (15) cambendazole; and (16) inhibitors of histone demethylase. In still another alternative, the eflornithine or pharmaceutically acceptable salt thereof is administered together with an agent that increases the ability of the eflornithine or pharmaceutically acceptable salt thereof to pass through the blood-brain barrier.

Another aspect of the present invention is a pharmaceutical composition for the treatment of glioma comprising:
(1) a therapeutically effective quantity of eflornithine or a pharmaceutically acceptable salt thereof;
(2) optionally, a therapeutically effective quantity of at least one additional agent that can be used together with eflornithine or a pharmaceutically acceptable salt thereof; and
(3) a pharmaceutically acceptable carrier;
wherein the composition is administered to reduce the rate of mutation of the glioma to reduce the progression of the glioma, wherein the glioma is temozolomide recurrent/ refractory anaplastic astrocytoma.

Additional agents are as described above. The pharmaceutical composition can be formulated for oral administration or administration by injection.

Conventional pharmaceutically acceptable carriers are known in the art and include, but are not limited to, a sugar, a solvent, a thickening agent, an emulsifying agent, a diluent, a sweetener, a wetting agent, an organic acid, a coloring agent, a flavoring agent, and a preservative.

### DETAILED DESCRIPTION OF THE INVENTION

In general, this invention is directed to the treatment of temozolomide recurrent/refractory anaplastic astrocytoma patients with eflornithine ("DFMO").

Neutral non-proteinogenic amino acids that are also AA T6 transporter substrates (such as DFMO) can extend life of cancer patients by inhibiting progression of DNA mutations caused by chemotherapy agents.

The basis of the invention, in general, is as follows: (1) chemotherapy agents cause DNA mutations; (2) DFMO interrupts cellular proliferation and differentiation; and (3) by interrupting cellular proliferation and differentiation in cancer cells, DFMO inhibits mutations and thus constrains progression of cancer. Therefore, since chemotherapy agents cause DNA mutations, use of DFMO with these agents inhibits those mutations and thus improves survival of cancer patients.

Eflornithine occurs in two enantiomeric forms: D-eflornithine and L-eflornithine. D-eflorninthine is shown in Formula (Ia), below. L-eflornithine is shown in Formula (lb), below. 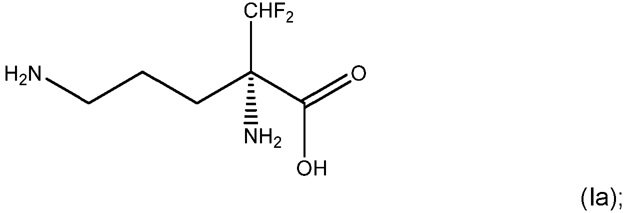 and

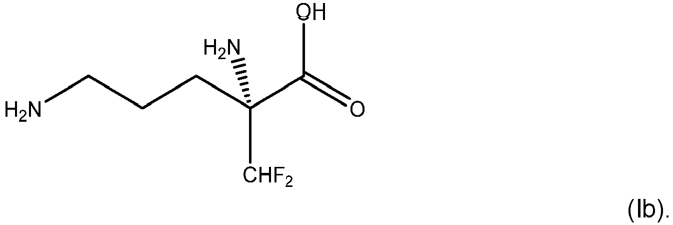

Typically, eflornithine is administered as the racemic mixture of D-eflornithine and L-eflornithine. However, eflornithine can also be administered in a mixture in which the D-eflornithine is relatively enriched with respect to the L-eflornithine, or in a pure or substantially pure preparation of D-eflornithine.

Eflornithine is a structural analog of the amino acid L-ornithine (shown below as Formula (II)

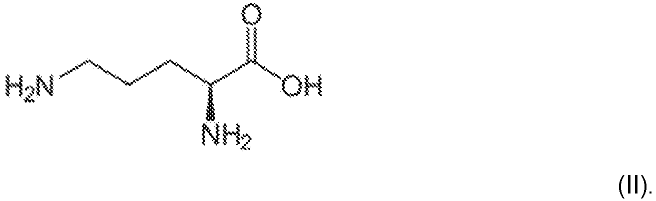

It is known that catalysis by ornithine decarboxylase (ODC) is the rate-limiting step in polyamine synthesis. The pathway for polyamine synthesis begins with L-ornithine. This natural amino acid, although not normally incorporated into proteins, is part of the urea cycle which metabolizes arginine to ornithine and urea. Ornithine is converted by ornithine decarboxylase (ODC) to putrescine and CO₂ and is considered to be the rate-limiting step in the production of polyamines. With the addition of propylamine donated from S-adenosylmethionine, putrescine is converted to spermidine. Spermidine is then converted to spermine by spermine synthetase, again in association with the decarboxylation of S-adenosylmethionine. Putrescine, spermidine and spermine represent the three major polyamines in mammalian tissues. Polyamines are found in animal tissues and microorganisms and are known to play an important role in cell growth and proliferation. Although the mechanism of the action of eflornithine in treating glioma is believed to involve primarily the prevention of induction of mutation in tumor cells, the effect of eflornithine on the synthesis of polyamines may play a secondary role.

A number of derivatives and analogs of eflornithine are known in the art, and are described further below.

Eflornithine is an irreversible inhibitor of the enzyme ornithine decarboxylase (ODC) and was originally developed as a treatment for trypanosomiasis (1-3). It has also been studied as a treatment for a variety of cancers (4). Eflornithine can be administered either orally or by injection, such as intravenously or intraperitoneally.

While it has been established that the primary action of eflornithine is to inhibit ODC activity and, thereby, the production of putrescine from ornithine, its pleiotropic effect as an anticancer agent has not been fully realized or understood at this time. Many actions have been proposed to explain the effectiveness of eflornithine on tumor cells (4-6). It has been long-held dogma that since polyamines (putrescine, spermidine, and spermine) play essential roles in DNA and RNA function that inhibition of ODC would inhibit tumor growth, and possibly tumor cell migration. Eflornithine can also reduce the effect of chemical carcinogens on colonic, skin, and bladder tissues and cell lines and in clinical settings (4, 6).

However, over the past several years new insights have come forward that suggests a different antitumor activity for eflornithine against CNS gliomas than had previously been realized or suggested. The invention is directed to this new basis for antitumor activity. Specifically, we assert that a major anticancer benefit of eflornithine rests with its ability to modulate (downregulate) mutation in slowly growing infiltrative gliomas (WHO Grade II and III tumors). By reducing the occurrence and/or number of mutations, we believe that tumor growth in the patient will cease and/or slow because the mutational-activated drivers of cancer progression and transformation to the more malignant glioblastoma (WHO Grade IV) will fail to occur or be less numerous over time.

A phase 3 randomized trial of adjuvant chemotherapy of eflornithine-PCV versus PCV (procarbazine/CCNU/vincristine) in anaplastic glioma patients (7) provides evidence for this hypothesis. That study showed that eflornithine-PCV chemotherapy produced a shift in the progression-free survival (PFS) hazard function compared to PCV chemotherapy that lasted about 1-1.5 years after the eflornithine-PCV stopped. From that point forward, the PFS and overall survival (OS) curves remained parallel, but did not cross, for over a decade (7,8). The hypothesis includes the probability that eflornithine protected against progression of anaplastic gliomas (especially anaplastic astrocytoma) to a more malignant phenotype, such as glioblastoma. Additional support comes from recent studies in neuroblastoma cells that found that eflornithine could increase two intracellular proteins, p21 and p27kip-1, and thereby arrest cell division between G1 and the initiation of mitosis (9,10).

Taken together, these two observations suggest that because eflornithine can be safely administered orally for 2 weeks every 3 weeks for years at a time, produce G1 arrest, and increase in intracellular p21 and p27kip-1 it is highly likely that eflornithine will reduce mutation-rates in glioma tumor cells in situ and, thereby, provide new and unexpected effects on the transformation of low- (WHO Grade II) and mid-grade (WHO Grade III) gliomas to glioblastoma (WHO Grade IV). This approach will, by its action, limit mutation and produce long-term survival gains for patients with these tumors as was shown in the clinical trial (7,8). It also suggests that treatment with eflornithine should continue for years in patients with low- and mid-grade gliomas. The increase in intracellular p21 and p27kip-1 induced by the administration of eflornithine is associated with the suppression of mutation by this agent, which has the clinical consequences of preventing or delaying the progression of the glioma to a higher grade.

Additional results support this hypothesis (11). These results show the importance of mutation to transformation of low- and mid-grade gliomas to more malignant tumor grades. The premise of this study was that therapies for recurrent or progressive gliomas failed because the genomic alterations driving the growth of recurrences were distinct from those in the initial tumor. In this study, the exomes of 23 initial low-grade gliomas and recurrent tumors resected from the same patients were sequenced. It was found that the three genes most commonly mutated in Grade 2 glioma at initial diagnosis were: *IDH1* in 100% (23/23), *TP53* in 83% (19/23), and *ATRX* in 78% (18/23) in the cohort studied. The next most commonly mutated gene, *SMARCA4,* was identified in 13% (3/23) of the initial tumors in this cohort. They also found 13 additional genes that could be identified in 9% (2/23) of the cohort. Interestingly, in 43% of cases, at least half of the mutations in the initial tumor were undetected at tumor recurrence/progression, including driver mutations in *TP53, ATRX, SMARCA4,* and *BRAF,* suggesting that recurrent tumors may be seeded by cells derived from the initial tumor at a very early stage of their evolution. This emphasizes the importance of early treatment for these tumors. Of additional interest also was the observation that tumors from 6 of 10 patients treated with adjuvant temozolomide (TMZ) chemotherapy followed an alternative evolutionary path to high-grade glioma: these tumors showed hypermutation and harbored driver mutations in the RB and AKT-mTOR pathways that bore the signature of TMZ-induced mutagenesis. These studies extended earlier observations and studies of primary GBMs (12,13), unpaired recurrent tumors (14), and a cell culture model (15).

Given that all WHO Grade II and III gliomas will follow a path of mutation if they recur or progress, one logical approach to control of these gliomas would be to mitigate the rate and extent of mutations these tumors can express. While causal proof that eflornithine impaired mutation rates in patients with anaplastic gliomas treated (7,8) is lacking at present, as discussed previously, circumstantial evidence favors a role of eflornithine in mitigating tumor cell mutations. To recall the facts, eflornithine can produce G1-arrest in neuroblastoma, a neuroectodermal tumor like glioma, by increasing intracellular p21 and p27kip-1 proteins (9,10) and, without much doubt, impact tumor cell mutation rates. It was previously found (16) that topical eflornithine treatment of biopsied skin actinic keratosis reduced the percentage of p53-positive cells (22%; P = 0.04) but not the frequency of p53 mutations compared to the placebotreated skin.

This hypothesis can be evaluated by experiments that could prove or support the conclusion that eflornithine can reduce mutation rates in low- and mid-grade gliomas. However, these tumors generally grow poorly outside of the human host. An alternative would be to use one or more conventional cell lines that grow in three-dimensional culture and that do not have a large number of mutations at the outset.

Another approach would be to use Big Blue Rat-2 cells in a similar approach to that used to evaluate the mutagenic potential of TMZ (15). In addition to looking at DNA adducts they looked at lacl mutations in Big Blue Rat-2 cells and found a dose-dependent increase in lacl mutation frequency from 9.1 to 48.9 and 89.7 treated with TMZ at 0, 0.5, or 1 mM TMZ. Sequence analysis of the lacl mutants from the TMZ treatment group demonstrated that they were GC-->AT transitions at non-CpG sites, which is significantly different from the mutation spectrum observed in the control treatment group. It is thus conceivable that one could treat Big Blue Rat-2 cells with eflornithine after a defined dose and duration of TMZ exposure has been given to initiate the mutation cascade.

Another possible approach would be take a slowly developing IC rodent tumor that kills animals over a time period of about 6 months and look into the mutations that occur at 2, 4, and 6 months and divide mice into two groups. Group 1 consisted of eflornithine (1.5-2%) in drinking water for 3-weeks/4-weeks for months 2-6 versus Group 2 which did not receive eflornithine. This approach using ∼500 gene sequencing on each tumor tissue sample obtained at euthanasia at 2, 4, and 6 months after tumor implantation might be sufficient to provide the information and proof of eflornithine effectiveness on mutation frequency.

Techniques previously described (23,24) for growing glioma or adenocarcinoma cells in three-dimensional culture and then evaluating the effect of treatment with single agents or drug combinations can be used. These techniques were originally developed to rapidly isolate phosphoproteins from 3-dimensional cultures under conditions of serum starvation or hypoxia (25,26), but these techniques will work equally well for DNA and RNA isolates. One approach will be to use the monofunctional alkylating agent, temozolomide, to produce mutation and then to give eflornithine afterwards at 2 doses and 2 times to determine how well eflornithine reduces the mutation frequency of the tumor cells. Temozolomide and eflornithine can be studied in such three-dimensional cultures with good results (23,24) so that it is expected that it would be possible to establish culture conditions for looking at mutation frequencies using 500-800 gene chip arrays at each time and dose point.

Water-soluble salts of eflornithine with polycations such as polycationic carbohydrates (chitosan, water-soluble chitosan derivative, or a salt thereof) or a polyaminoacid, a polyamine, a polypeptide, a basic polymer, or a quaternary ammonium compound are disclosed in United States Patent Application Publication No. 2002/0019338 by Hebert. All pharmaceutically acceptable salt forms, hydrates, and solvates of eflornithine can be used in methods and compositions of the present invention.

United States Patent Application Publication No. 2002/0110590 by Shaked et al. discloses formulations for the administration of eflornithine, including a core having a rapid release DFMO-containing granules and a slow release granule and an outer layer surrounding the core comprising a pH responsive coating.

United States Patent No. 9,034,319 to Teichberg et al. discloses the use of eflornithine together with an agent which reduces blood glutamate levels and enhances brain to blood glutamate efflux. The agent that reduces blood glutamate levels and enhances brain to blood glutamate efflux can be: (1) a transaminase that can be selected from the group consisting of glutamate oxaloacetate transaminase, glutamate pyruvate transaminase, acetylornithine transaminase, ornithine-oxo-acid transaminase, succinyldiaminopimelate transaminase, 4-aminobutyrate transaminase, (s)-3-amino-2-methylpropionate transaminase, 4-hydroxyglutamate transaminase, diiodotyrosine transaminase, thyroid-hormone transaminase, tryptophan transaminase, diamine transaminase, cysteine transaminase, L-Lysine 6-transaminase, histidine transaminase, 2-aminoadipate transaminase, glycine transaminase, branched-chain-amino-acid transaminase, 5-aminovalerate transaminase, dihydroxyphenylalanine transaminase, tyrosine transaminase, phosphoserine transaminase, taurine transaminase, aromatic-amino-acid transaminase, aromatic-amino-acid-glyoxylate transaminase, leucine transaminase, 2-aminohexanoate transaminase, ornithine(lysine) transaminase, kynurenine-oxoglutarate transaminase, D-4-hydroxyphenylglycine transaminase, cysteine-conjugate transaminase, 2,5-diaminovalerate transaminase, histidinol-phosphate transaminase, diaminobutyrate-2-oxoglutarate transaminase, and udp-2-acetamido-4-amino-2,4,6-trideoxyglucose transaminase; (2) a glutamate dehydrogenase; (3) a glutamate decarboxylase; (4) a glutamate-ethylamine ligase; (5) a transferase that can be selected from the group consisting of glutamate N-acetyltransferase and adenylyltransferase; (6) an aminomutase that can be glutamate-1-semialdehyde 2,1-aminomutase; and (7) a racemase. The enzyme can be used with a cofactor.

United States Patent No. 6,277,411 to Shaked et al. discloses preparations comprising a capsule, tablet or other dosage form containing a core of different types of eflornithine.

Accordingly, as detailed further below, one aspect of the present invention is a method for the treatment of glioma comprising the step of administering a therapeutically effective quantity of eflornithine or a pharmaceutically acceptable salt thereof to a subject with glioma in order to reduce the rate of mutation of the glioma to reduce the progression of the glioma, wherein the glioma is temozolomide recurrent/ refractory anaplastic astrocytoma.

Eflornithine or a pharmaceutically acceptable salt thereof as described above can be used together with other agents. Pharmaceutically acceptable salt forms, hydrates, and solvates of eflornithine can be used individually or together with other agents.

United States Patent No. 9,150,495 to Phanstiel, IV discloses the use of eflornithine together with a polyamine transporter selective compound, including aromatic hydrocarbons di-substituted with a polyamine.

United States Patent No. 9,072,778 to Bachmann discloses the use of eflornithine together with SAM486A (an S-adenosylmethionine decarboxylase inhibitor, 4-(aminoiminomethyl)-2,3-dihydro-1H-inden-1-one-diaminomethylenehydrazone), a verinoid, and an antineoplastic drug.

United States Patent No. 8,597,904 to Bachmann et al. discloses use of eflornithine together with glidobactin, syringolin, and other syrbactin compounds.

United States Patent No. 7,655,678 to Gupta et al. discloses the use of eflornithine together with celecoxib. United States Patent Application Publication No. 2003/0203956 by Masterrer discloses the use of eflornithine with a cyclooxygenase-2 inhibitor selected from the group consisting of lumiracoxib, celecoxib, rofecoxib, etoricoxib, valdecoxib, parecoxib, and deracoxib. Similarly, United States Patent No. 6,258,845 to Gerner et al. discloses the use of eflornithine together with the non-steroidal anti-inflammatory sulindac.

United States Patent No. 7,432,302 to Burns et al. discloses the use of polyamine transport inhibitors together with eflornithine. The polyamine transport inhibitors can be compounds of structure R-X-L-polyamine wherein R is a straight or branched C₁₀-C₅₀ saturated or unsaturated aliphatic, carboxyalkyl, carbalkoxyalkyl, or alkoxy; a C₁-C₈ alicyclic moiety; a single or multiring aryl substituted or unsubstituted aliphatic; and aliphatic-substituted or unsubstituted single or multiring aromatic; a single or multiring heterocyclic; a single or multiring heterocyclic aliphatic; an aryl sulfonyl; X is --CO--, --SO₂--, or --CH₂--; and L is a covalent bond or a naturally occurring amino acid, lysine, ornithine, or 2,4-diaminobutyric acid.

United States Patent No. 7,425,579 to Poulin et al. discloses the use of polyamine transport inhibitors together with eflornithine. The polyamine transport inhibitors can be compounds of Formula (PT-I) or (PT-II): 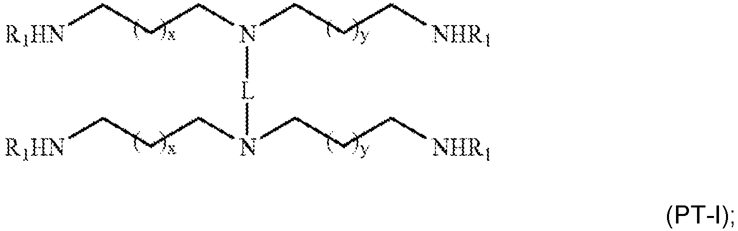 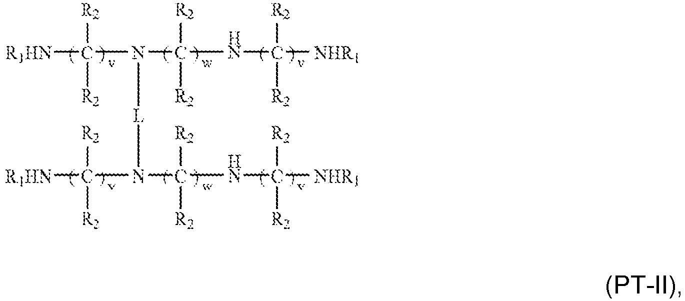 wherein: L is a linker; R₁ is hydrogen, methyl, ethyl, or propyl; R₂ is hydrogen or methyl; 0<x<3; 0<y<3; 2<v<5; and 2<w<8.

United States Patent No. 7,208,528 to Vermeulin et al. discloses the use of polyamine transport inhibitors together with eflornithine. The polyamine transport inhibitors can be an N¹-monosubstituted polyamine analog or derivative of Formula (PT-III)

R-CO-NH-(CH₂)₃-NH-CH₂)₄-NH-(CH₂)₃-NH₂ (PT-III),

wherein: R is selected from a D or L amino acid; D or L ornithine, an alicyclic, a single or multi-ring aromatic; aliphatic-substituted single or multi-ring aromatic; and a substituted or unsubstituted, single or multi-ring heterocyclic and wherein when R is a substituted single or multi-ring heterocyclic, heterocyclic is substituted with at least one member of the group consisting of: OH, halogen, NO₂, NH₂, NH(CH₂)ₙCH₃, N((CH₂)ₙCH₃)₂, CN, (CH₂)ₙCH₃, O(CH₂)ₙCH₃, S(CH₂)ₙCH₃, NHCO(CH₂)ₙCH₃, or O(CF₂)ₙCF₃, COO(CH₂)ₙCH₃, wherein n is 0-10.

United States Patent No. 7,160,923 to Vermeulin et al. discloses the use of polyamine transport inhibitors together with eflornithine. The polyamine transport inhibitors can have the formula R₁-X-R₂, wherein R₁-X- is of the formula R-NH-CR'R"-CO-; wherein NH-CR'R"-CO- is a D- or L-form of valine, asparagine, or glutamine, or the D-form of lysine or arginine; wherein R" is H, CH₃, CH₂CH₃, or CHF₂; where R is H or a head group selected from the group consisting of a straight or branched C₁-C₁₀ aliphatic, alicyclic, single or multiring aromatic, single or multiring aryl substituted aliphatic, aliphatic-substituted single or multiring aromatic, a single or multiring heterocyclic, a single or multiring heterocyclic-substituted aliphatic and an aliphatic-substituted aromatic; and wherein R₂ is a polyamine.

United States Patent No. 7,144,920 to Burns et al. discloses polyamine analogs that induce antizyme activity and inhibit polyamine transporter activity and that can be used with eflornithine, including compounds of Formula (PT-IV): 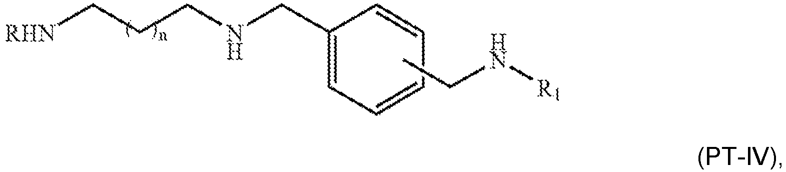 wherein: n can be 0 to 8 and the aminomethyl functionality can be ortho, meta or para substituted, R is hydrogen, 2-aminoethyl, 3-aminopropyl, 4-aminobutyl, 5-aminopentyl, 6-aminohexyl, 7-aminoheptyl, or 8-aminooctyl and R₁ is hydrogen and wherein the polyamine is non-symmetrical.

United States Patent No. 7,094,808 to Bergeron, Jr. discloses polyamine transport inhibitors of Formula (PT-V): 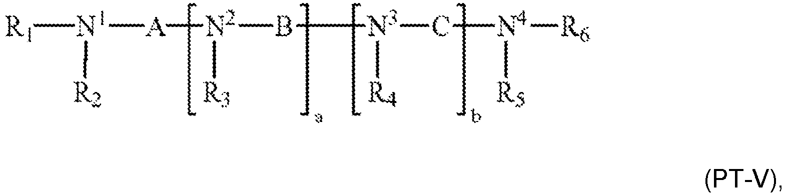 wherein: R₁-R₆ may be the same or different and are alkyl, aryl, aryl alkyl, or cycloalkyl, optionally having an alkyl chains interrupted by at least one etheric oxygen atom, or hydrogen; N¹, N², N³ and N⁴ are nitrogen atoms capable of protonation at physiological pH's; a and b may be the same or different and are integers from 1 to 4; A, B and C may be the same or different and are bridging groups which effectively maintain the distance between the nitrogen atoms such that the polyamine: (i) is capable of uptake by a target cell upon administration of the polyamine to a human or non-human animal; and (ii) upon uptake by the target cell, competitively binds via an electrostatic interaction between the positively charged nitrogen atoms to substantially the same biological counter-anions as the intra-cellular natural polyamines in the target cell; the polyamine, upon binding to the biological counter-anion in the cell, functions in a manner biologically different than the intracellular polyamines, the polyamine not occurring in nature.

United States Patent No. 7,030,126 to Ramesh et al. discloses the use of the polyamine analog N(1),N(11)-diethylnorspermine (DENSPM), which can be used with eflornithine, as a polyamine synthesis inhibitor.

United States Patent No. 6,963,010 to Burns et al. discloses the use of hydrophobic polyamine analogs that can be used with eflornithine. These analogs include analogs of Formulas (PT-VI), (PT-VII), (PT-VIII), and (PT-IX):

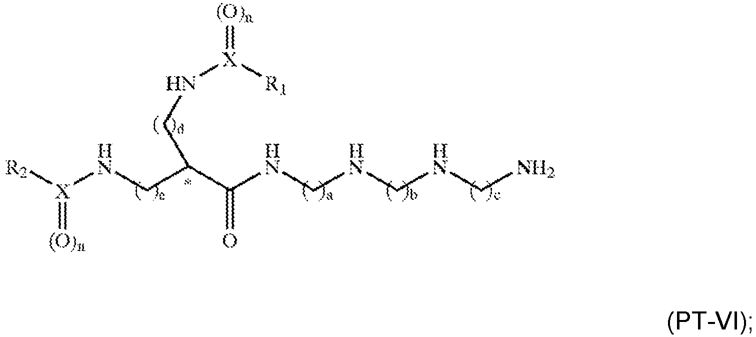

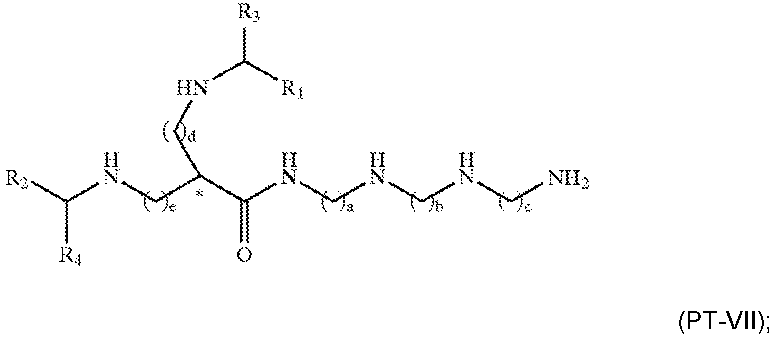

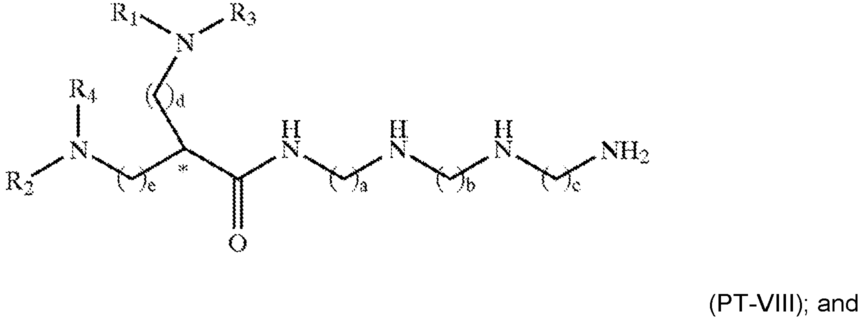

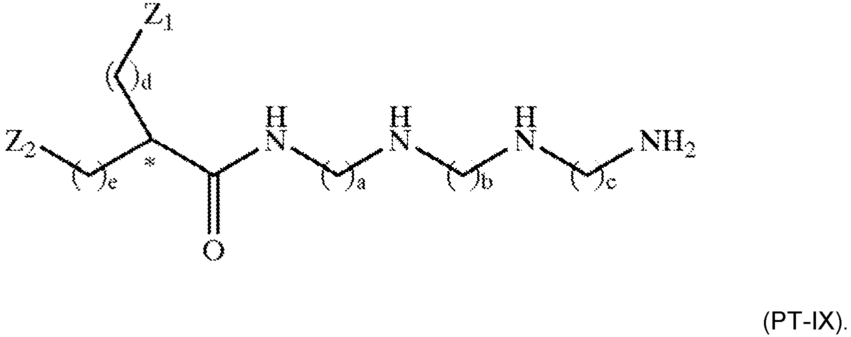

In compounds of Formula (PT-VI): a, b, and c independently range from 1 to 10; d and e independently range from 0 to 30; each X is independently either a carbon (C) or sulfur (S) atom, and R₁ and R₂ are independently selected from H or from the group of a straight or branched C₁-C₅₀ saturated or unsaturated aliphatic, carboxyalkyl, carbalkoxyalkyl, or alkoxy; a C₁-C₈ alicyclic; a single or multiring aryl substituted or unsubstituted aliphatic; an aliphatic-substituted or unsubstituted single or multiring aromatic; a single or multiring heterocyclic; a single or multiring heterocyclic aliphatic; a C₁-C₁₀ alkyl; an aryl sulfonyl; or cyano; or each of R₁ X{O}ₙ -- and R₂ X{O}ₙare independently replaced by H; wherein * denotes a chiral carbon position; and wherein if X is C, then n is 1; if X is S, then n is 2; and if X is C, then the XO group may be CH₂ such that n is 0.

In compounds of Formula (PT-VII): a, b, and c independently range from 1 to 10 and d and e independently range from 0 to 30; and R₁, R₂, R₃, and R₄ may be the same or different and are independently selected from H or from the group of a straight or branched C₁-C₅₀ saturated or unsaturated aliphatic, carboxyalkyl, carbalkoxyalkyl, or alkoxy; a C₁-C₈ alicyclic; a single or multiring aryl substituted or unsubstituted aliphatic; an aliphatic-substituted or unsubstituted single or multiring aromatic; a single or multiring heterocyclic; a single or multiring heterocyclic aliphatic; a C₁-C₁₀ alkyl; an aryl sulfonyl; or cyano.

In compounds of Formula (PT-VIII): a, b, and c independently range from 1 to 10 and d and e independently range from 0 to 30; and R₁, R₂, R₃, and R₄ may be the same or different and are independently selected from H or from the group of a straight or branched C₁-C₅₀ saturated or unsaturated aliphatic, carboxyalkyl, carbalkoxyalkyl, or alkoxy; a C₁-C₈ alicyclic; a single or multiring aryl substituted or unsubstituted aliphatic; an aliphatic-substituted or unsubstituted single or multiring aromatic; a single or multiring heterocyclic; a single or multiring heterocyclic aliphatic; a C₁-C₁₀ alkyl; an aryl sulfonyl; or cyano.

In compounds of Formula (PT-IX): a, b, and c independently range from 1 to 10 and d and C independently range from 0 to 30; and wherein Z₁ is NR₁R₃ and Z₂ is selected from --R₁, --CHR₁R₂ or --CR₁R₂ R₃ or Z₂ is NR₂R₄ and Z₁ is selected from --R₁, --CHR₁R₂ or --CR₁R₂R₃, wherein R₁, R₂, and R₃ may be the same or different and are independently selected from H or from the group of a straight or branched C₁-C₅₀ saturated or unsaturated aliphatic, carboxyalkyl, carbalkoxyalkyl, or alkoxy; a C₁-C₈ alicyclic; a single or multiring aryl substituted or unsubstituted aliphatic; an aliphatic-substituted or unsubstituted single or multiring aromatic; a single or multiring heterocyclic; a single or multiring heterocyclic aliphatic; a C₁-C₁₀ alkyl; an aryl sulfonyl; or cyano.

United States Patent No. 6,872,852 to Burns et al. discloses polyamine analogs that can be used with eflornithine, including compounds of the formula R₁-X-R₂, wherein R₁ and R₂ are independently H or a moiety selected from the group consisting of a straight or branched C₁-C₁₀ aliphatic, alicyclic, single or multiring aromatic, single or multi-ring aryl substituted aliphatic, aliphatic-substituted single or multiring aromatic, a single or multiring heterocyclic, a single or multi-ring heterocyclic-substituted aliphatic and an aliphatic-substituted aromatic, and halogenated forms thereof; and X is a polyamine with two terminal amino groups, --(CH₂)₃--NH--, or --CH₂--Ph--CH₂--.

United States Patent No. 6,646,149 to Vermeulen et al. discloses polyamine analogs that inhibit polyamine transporter activity and can be used with eflornithine, including compounds of the formula R₁-X-R₂, wherein R₁ and R₂ are each a polyamine or an analog or derivative of a polyamine and X is a linker moiety connecting the two polyamine moieties.

United States Patent No. 6,392,098 to Frydman et al. discloses conformationally restricted polyamine analogs that can be used with eflornithine, including compounds of formula E-NH-D-NH-B-A-B-NH-D-NH-E, wherein: A is selected from the group consisting of C₂-C₆ alkenyl and C₃-C₆ cycloalkyl, cycloalkenyl, and cycloaryl; B is independently selected from the group consisting of a single bond and C₁-C₆ alkyl and alkenyl; D is independently selected from the group consisting of C₁-C₆ alkyl and alkenyl, and C₃-C₆ cycloalkyl, cycloalkenyl, and cycloaryl; E is independently selected from the group consisting of H, C₁-C₆ alkyl and alkenyl.

United States Patent No. 6,083,496 to Poulin et al. discloses inhibitors of polyamine transport that can be used with eflornithine including synthetic derivatives of a dimer of an original polyamine, wherein the original polyamine is modified to comprise an amido group immediately linked to a carbon atom of the original polyamine and being located between two internal atoms, the dimer being linked together by a spacer side chain anchored to the amido group of each monomer.

United States Patent No. 5,880,161 to Basu et al. discloses polyamine analogs that can be used with eflornithine, including molecules having a formula R₁-NH-(CH₂)_{w}-NH-(CH₂)ₓ-NH-(CH₂)_{y}-NH-(CH₂)_{z}-NH-R₂, wherein R₁ and R₂ are hydrocarbon chains of 1 to 5 carbons and w, x, y, and z are integers of 1 to 10; one preferred molecule is N¹,N¹⁹-bis-(ethylamino)-5,10,15-triazanonadecane.

United States Patent No. 5,374,658 to Lau discloses use of oxidized polyamines including N,N'-bis-(3-propionaldehyde)-1,4-diaminobutane (spermine bisaldehyde) together with eflornithine.

United States Patent No. 4,952,585 to Sunkara et al. discloses the use of eflornithine with esters of castanospermine. Similarly, United States Patent No. 4,792,558 to Sunkara et al. discloses the use of eflornithine with castanospermine.

United States Patent No. 4,925,835 to Heston discloses aziridinyl putrescine compounds such as 1-(4-aminobutyl)aziridine that can be used together with eflornithine.

United States Patent No. 4,499,072 to Sunkara et al. discloses the use of eflornithine with interferon.

United States Patent Application Publication No. 2010/0076009 by Towner et al. discloses the use of eflornithine with 2,4-disulfonyl phenyl tert-butyl nitrone (2,4-ds-PBN) in the treatment of glioma.

United States Patent Application Publication No. 2015/0094336 by Zeldis discloses the use of eflornithine with 3-(4-amino-1-oxo-1,3-dihydro-isoindol-2-yl)-piperidine-2,6-dione or thalidomide in the treatment of glioma.

United States Patent Application Publication No. 2010/0285012 by Dunn, Jr. et al. discloses the use of eflornithine with N-2-pyridinyl-2-pyridinecarbothioamide or cambendazole in the treatment of glioma.

United States Patent Application Publication No. 2013/0197088 by Casero, Jr. et al. discloses the use of eflornithine with inhibitors of histone demethylase, including oligoamines and polyamines, for the treatment of malignancies.

United States Patent Application Publication No. 2015/0050299 by Burns et al. discloses the use of eflornithine together with one of a number of polyamine transport inhibitors, including AMXT 1426, AMXT 1501, AMXT 1505, and AMXT 1569.

United States Patent Application Publication No. 2008/0027023 by Ellervik et al. discloses the use of eflornithine together with aryl substituted xylopyranoside derivatives.

United States Patent Application Publication No. 2015/0017231 by Phanstiel, IV et al. discloses the use of eflornithine together with polyamine transport inhibitors with increased stability. The polyamine transport inhibitors are di-substituted aryl polyamine compounds with the structure R'HN-(CH₂)ₓ-NH-(CH₂)_{y-}-NH-CH₂-R-CH₂--NH--(CH₂)ₓₓ--NH--(CH₂)_{yy}--NHR" wherein R is selected from the group consisting of anthracene, naphthalene, and benzene; wherein R' and R" are independently selected from the group consisting of H and an alkyl group; and wherein x, xx, y, and yy are independently selected from the group consisting of 3 and 4.

The compounds described above can optionally be further substituted. In general, for optional substituents at saturated carbon atoms such as those that are part of the structures of the compounds described above, the following substituents can be employed: C₆-C₁₀ aryl, heteroaryl containing 1-4 heteroatoms selected from N, O, and S, C₁-C₁₀ alkyl, C₁-C₁₀ alkoxy, cycloalkyl, F, amino (NR¹R²), nitro, -SR, -S(O)R, - S(O₂)R, -S(O₂)NR¹R², and -CONR¹R², which can in turn be optionally substituted. Further descriptions of potential optional substituents are provided below.

Optional substituents as described above that are present in derivatives of eflornithine (not claimed) do not substantially affect the activity of the derivative or the stability of the derivative, particularly the stability of the derivative in aqueous solution. Definitions for a number of common groups that can be used as optional substituents are provided below; however, the omission of any group from these definitions cannot be taken to mean that such a group cannot be used as an optional substituent as long as the chemical and pharmacological requirements for an optional substituent are satisfied.

As used herein, the term "alkyl" refers to an unbranched, branched, or cyclic saturated hydrocarbyl residue, or a combination thereof, of from 1 to 12 carbon atoms that can be optionally substituted; the alkyl residues contain only C and H when unsubstituted. Typically, the unbranched or branched saturated hydrocarbyl residue is from 1 to 6 carbon atoms, which is referred to herein as "lower alkyl." When the alkyl residue is cyclic and includes a ring, it is understood that the hydrocarbyl residue includes at least three carbon atoms, which is the minimum number to form a ring. As used herein, the term "alkenyl" refers to an unbranched, branched or cyclic hydrocarbyl residue having one or more carbon-carbon double bonds. As used herein, the term "alkynyl" refers to an unbranched, branched, or cyclic hydrocarbyl residue having one or more carbon-carbon triple bonds; the residue can also include one or more double bonds. With respect to the use of "alkenyl" or "alkynyl," the presence of multiple double bonds cannot produce an aromatic ring. As used herein, the terms "hydroxyalkyl," "hydroxyalkenyl," and "hydroxyalkynyl," respectively, refer to an alkyl, alkenyl, or alkynyl group including one or more hydroxyl groups as substituents; as detailed below, further substituents can be optionally included. As used herein, the term "aryl" refers to a monocyclic or fused bicyclic moiety having the well-known characteristics of aromaticity; examples include phenyl, naphthyl, fluorenyl, and indenyl, which can be optionally substituted. As used herein, the term "hydroxyaryl" refers to an aryl group including one or more hydroxyl groups as substituents; as further detailed below, further substituents can be optionally included. As used herein, the term "heteroaryl" refers to monocyclic or fused bicylic ring systems that have the characteristics of aromaticity and include one or more heteroatoms selected from O, S, and N. The inclusion of a heteroatom permits aromaticity in 5-membered rings as well as in 6-membered rings. Typical heteroaromatic systems include monocyclic C₅-C₆ heteroaromatic groups such as pyridyl, pyrimidyl, pyrazinyl, thienyl, furanyl, pyrrolyl, pyrazolyl, thiazolyl, oxazolyl, triazolyl, triazinyl, tetrazolyl, tetrazinyl, and imidazolyl, as well as the fused bicyclic moieties formed by fusing one of these monocyclic heteroaromatic groups with a phenyl ring or with any of the heteroaromatic monocyclic groups to form a C₈-C₁₀ bicyclic group such as indolyl, benzimidazolyl, indazolyl, benzotriazolyl, isoquinolyl, quinolyl, benzothiazolyl, benzofuranyl, pyrazolylpyridyl, quinazolinyl, quinoxalinyl, cinnolinyl, and other ring systems known in the art. Any monocyclic or fused ring bicyclic system that has the characteristics of aromaticity in terms of delocalized electron distribution throughout the ring system is included in this definition. This definition also includes bicyclic groups where at least the ring that is directly attached to the remainder of the molecule has the characteristics of aromaticity, including the delocalized electron distribution that is characteristic of aromaticity. Typically the ring systems contain 5 to 12 ring member atoms and up to four heteroatoms, wherein the heteroatoms are selected from the group consisting of N, O, and S. Frequently, the monocyclic heteroaryls contain 5 to 6 ring members and up to three heteroatoms selected from the group consisting of N, O, and S; frequently, the bicyclic heteroaryls contain 8 to 10 ring members and up to four heteroatoms selected from the group consisting of N, O, and S. The number and placement of heteroatoms in heteroaryl ring structures is in accordance with the well-known limitations of aromaticity and stability, where stability requires the heteroaromatic group to be stable enough to be exposed to water at physiological temperatures without rapid degradation. As used herein, the term "hydroxheteroaryl" refers to a heteroaryl group including one or more hydroxyl groups as substituents; as further detailed below, further substituents can be optionally included. As used herein, the terms "haloaryl" and "haloheteroaryl" refer to aryl and heteroaryl groups, respedively, substituted with at least one halo group, where "halo" refers to a halogen selected from the group consisting of fluorine, chlorine, bromine, and iodine, typically, the halogen is selected from the group consisting of chlorine, bromine, and iodine; as detailed below, further substituents can be optionally included. As used herein, the terms "haloalkyl," "haloalkenyl," and "haloalkynyl" refer to alkyl, alkenyl, and alkynyl groups, respectively, substituted with at least one halo group, where "halo" refers to a halogen selected from the group consisting of fluorine, chlorine, bromine, and iodine, typically, the halogen is selected from the group consisting of chlorine, bromine, and iodine; as detailed below, further substituents can be optionally included.

As used herein, the term "optionally substituted" indicates that the particular group or groups referred to as optionally substituted may have no non-hydrogen substituents, or the group or groups may have one or more non-hydrogen substituents consistent with the chemistry and pharmacological activity of the resulting molecule. If not otherwise specified, the total number of such substituents that may be present is equal to the total number of hydrogen atoms present on the unsubstituted form of the group being described; fewer than the maximum number of such substituents may be present. Where an optional substituent is attached via a double bond, such as a carbonyl oxygen (C=O), the group takes up two available valences on the carbon atom to which the optional substituent is attached, so the total number of substituents that may be included is reduced according to the number of available valences. As used herein, the term "substituted," whether used as part of "optionally substituted" or otherwise, when used to modify a specific group, moiety, or radical, means that one or more hydrogen atoms are, each, independently of each other, replaced with the same or different substituent or substituents.

Substituent groups useful for substituting saturated carbon atoms in the specified group, moiety, or radical include, but are not limited to, -Z^{a}, =O, -OZ^{b}, - SZ^{b}, =S⁻, -NZ^{c}Z^{c}, =NZ^{b}, =N-OZ^{b}, trihalomethyl, -CF₃, -CN, -OCN, -SCN, -NO, -NO₂, =N₂, -N₃, -S(O)₂Z^{b}, -S(O)₂NZ^{b}, -S(O₂)O⁻, -S(O₂)OZ^{b}, -OS(O₂)OZ^{b}, - OS(O₂)O⁻, -OS(O₂)OZ^{b}, -P(O)(O')₂, -P(O)(OZ^{b})(O⁻), -P(O)(OZ^{b})(OZ^{b}), -C(O)Z^{b}, -C(S)Z^{b}, -C(NZ^{b})Z^{b}, -C(O)O⁻, -C(O)OZ^{b}, -C(S)OZ^{b}, -C(O)NZ^{c}Z^{c}, - C(NZ^{b})NZ^{c}Z^{c}, -OC(O)Z^{b}, -OC(S)Z^{b}, -OC(O)O⁻, -OC(O)OZ^{b}, -OC(S)OZ^{b}, - NZ^{b}C(O)Z^{b}, -NZ^{b}C(S)Z^{b}, -NZ^{b}C(O)O⁻, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, -NZ^{b}C(NZ^{b})NZ^{c}Z^{c}, wherein Z^{a} is selected from the group consisting of alkyl, cycloalkyl, heteroalkyl, cycloheteroalkyl, aryl, arylalkyl, heteroaryl and heteroarylalkyl; each Z^{b} is independently hydrogen or Z^{a}; and each Z^{c} is independently Z^{b} or, alternatively, the two Z^{c}'s may be taken together with the nitrogen atom to which they are bonded to form a 4-, 5-, 6-, or 7-membered cycloheteroalkyl ring structure which may optionally include from 1 to 4 of the same or different heteroatoms selected from the group consisting of N, O, and S. As specific examples, -NZ^{c}Z^{c} is meant to include -NH₂, -NH-alkyl, -N-pyrrolidinyl, and -N-morpholinyl, but is not limited to those specific alternatives and includes other alternatives known in the art. Similarly, as another specific example, a substituted alkyl is meant to include - alkylene-O-alkyl, -alkylene-heteroaryl, -alkylene-cycloheteroaryl, -alkylene-C(O)OZ^{b}, -alkylene-C(O)NZ^{b}Z^{b}, and -CH₂-CH₂-C(O)-CH₃, but is not limited to those specific alternatives and includes other alternatives known in the art. The one or more substituent groups, together with the atoms to which they are bonded, may form a cyclic ring, including, but not limited to, cycloalkyl and cycloheteroalkyl.

Similarly, substituent groups useful for substituting unsaturated carbon atoms in the specified group, moiety, or radical include, but are not limited to, -Z^{a}, halo, -O⁻, -OZ^{b}, -SZ^{b}, -S⁻, -NZ^{c}Z^{c}, trihalomethyl, -CF₃, -CN, -OCN, -SCN, -NO, ⁻-NO₂, -N₃, -S(O)₂Z^{b}, -S(O₂)O⁻, -S(O₂)OZ^{b}, -OS(O₂)OZ^{b}, -OS(O₂)O⁻, - P(O)(O⁻)₂, -P(O)(OZ^{b})(O⁻), -P(O)(OZ^{b})(OZ^{b}), -C(O)Z^{b}, -C(S)Z^{b}, -C(NZ^{b})Z^{b}, - C(O)O⁻, -C(O)OZ^{b}, -C(S)OZ^{b}, -C(O)NZ^{c}Z^{c}, -C(NZ^{b})NZ^{c}Z^{c}, -OC(O)Z^{b}, -OC(S)Z^{b}, -OC(O)O⁻, -OC(O)OZ^{b}, -OC(S)OZ^{b}, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, and -NZ^{b}C(NZ^{b})NZ^{c}Z^{c}, wherein Z^{a}, Z^{b}, and Z^{c} are as defined above.

Similarly, substituent groups useful for substituting nitrogen atoms in heteroalkyl and cycloheteroalkyl groups include, but are not limited to, -Z^{a}, halo, -O⁻, -OZ^{b}, -SZ^{b}, -S⁻, -NZ^{c}Z^{c}, trihalomethyl, -CF₃, -CN, -OCN, -SCN, -NO, - NO₂, -S(O)₂Z^{b}, -S(O₂)O⁻, -S(O₂)OZ^{b}, -OS(O₂)OZ^{b}, -OS(O₂)O⁻, -P(O)(O⁻)₂, - P(O)(OZ^{b})(O⁻), -P(O)(OZ^{b})(OZ^{b}), -C(O)Z^{b}, -C(S)Z^{b}, -C(NZ^{b})Z^{b}, -C(O)OZ^{b}, - C(S)OZ^{b}, -C(O)NZ^{c}Z^{c}, -C(NZ^{b})NZ^{c}Z^{c}, -OC(O)Z^{b}, -OC(S)Z^{b}, -OC(O)OZ^{b}, - OC(S)OZ^{b}, -NZ^{b}C(O)Z^{b}, -NZ^{b}C(S)Z^{b}, -NZ^{b}C(O)OZ^{b}, -NZ^{b}C(S)OZ^{b}, - NZ^{b}C(O)NZ^{c}Z^{c}, -NZ^{b}C(NZ^{b})Z^{b}, and -NZ^{b}C(NZ^{b})NZ^{c}Z^{c}, wherein Z^{a}, Z^{b}, and Z^{c} are as defined above.

The compounds described herein may contain one or more chiral centers and/or double bonds and therefore, may exist as stereoisomers, such as double-bond isomers (i.e., geometric isomers such as E and Z), enantiomers or diastereomers. The invention includes each of the isolated stereoisomeric forms (such as the enantiomerically pure isomers, the E and Z isomers, and other alternatives for stereoisomers) as well as mixtures of stereoisomers in varying degrees of chiral purity or percentage of E and Z, including racemic mixtures, mixtures of diastereomers, and mixtures of E and Z isomers, unless a specific stereoisomer is specified. Accordingly, the chemical structures depicted herein encompass all possible enantiomers and stereoisomers of the illustrated compounds including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The invention includes each of the isolated stereoisomeric forms as well as mixtures of stereoisomers in varying degrees of chiral purity, including racemic mixtures. It also encompasses the various diastereomers. Other structures may appear to depict a specific isomer, but that is merely for convenience, and is not intended to limit the invention to the depicted isomer. When the chemical name does not specify the isomeric form of the compound, it denotes any one of the possible isomeric forms or mixtures of those isomeric forms of the compound. As stated above, eflornithine exists in two enantiomeric forms.

The compounds may also exist in several tautomeric forms, and the depiction herein of one tautomer is for convenience only, and is also understood to encompass other tautomers of the form shown. Accordingly, the chemical structures depicted herein encompass all possible tautomeric forms of the illustrated compounds. The term "tautomer" as used herein refers to isomers that change into one another with great ease so that they can exist together in equilibrium; the equilibrium may strongly favor one of the tautomers, depending on stability considerations. For example, ketone and enol are two tautomeric forms of one compound.

As used herein, the term "solvate" means a compound formed by solvation (the combination of solvent molecules with molecules or ions of the solute), or an aggregate that consists of a solute ion or molecule, i.e., a compound of the invention, with one or more solvent molecules. When water is the solvent, the corresponding solvate is "hydrate." Examples of hydrate include, but are not limited to, hemihydrate, monohydrate, dihydrate, trihydrate, hexahydrate, and other water-containing species. It should be understood by one of ordinary skill in the art that the pharmaceutically acceptable salt, and/or prodrug of the present compound may also exist in a solvate form. The solvate is typically formed via hydration which is either part of the preparation of the present compound or through natural absorption of moisture by the anhydrous compound of the present invention.

As used herein, the term "ester" means any ester of a present compound in which any of the --COOH functions of the molecule is replaced by a --COOR function, in which the R moiety of the ester is any carbon-containing group which forms a stable ester moiety, including but not limited to alkyl, alkenyl, alkynyl, cycloalkyl, cycloalkylalkyl, aryl, arylalkyl, heterocyclyl, heterocyclylalkyl and substituted derivatives thereof. The hydrolysable esters of the present compounds are the compounds whose carboxyls are present in the form of hydrolysable ester groups. That is, these esters are pharmaceutically acceptable and can be hydrolyzed to the corresponding carboxyl acid *in vivo.*

In addition to the substituents described above, alkyl, alkenyl and alkynyl groups can alternatively or in addition be substituted by C₁-C₈ acyl, C₂-C₈ heteroacyl, C₆-C₁₀ aryl, C₃-C₈ cycloalkyl, C₃-C₈ heterocyclyl, or C₅-C₁₀ heteroaryl, each of which can be optionally substituted. Also, in addition, when two groups capable of forming a ring having 5 to 8 ring members are present on the same or adjacent atoms, the two groups can optionally be taken together with the atom or atoms in the substituent groups to which they are attached to form such a ring.

"Heteroalkyl," "heteroalkenyl," and "heteroalkynyl" and the like are defined similarly to the corresponding hydrocarbyl (alkyl, alkenyl and alkynyl) groups, but the 'hetero' terms refer to groups that contain 1-3 0, S or N heteroatoms or combinations thereof within the backbone residue; thus at least one carbon atom of a corresponding alkyl, alkenyl, or alkynyl group is replaced by one of the specified heteroatoms to form, respectively, a heteroalkyl, heteroalkenyl, or heteroalkynyl group. For reasons of chemical stability, it is also understood that, unless otherwise specified, such groups do not include more than two contiguous heteroatoms except where an oxo group is present on N or S as in a nitro or sulfonyl group.

While "alkyl" as used herein includes cycloalkyl and cycloalkylalkyl groups, the term "cycloalkyl" may be used herein to describe a carbocyclic non-aromatic group that is connected via a ring carbon atom, and "cycloalkylalkyl" may be used to describe a carbocyclic non-aromatic group that is connected to the molecule through an alkyl linker.

Similarly, "heterocyclyl" may be used to describe a non-aromatic cyclic group that contains at least one heteroatom (typically selected from N, O and S) as a ring member and that is connected to the molecule via a ring atom, which may be C (carbon-linked) or N (nitrogen-linked); and "heterocyclylalkyl" may be used to describe such a group that is connected to another molecule through a linker. The heterocyclyl can be fully saturated or partially saturated, but non-aromatic. The sizes and substituents that are suitable for the cycloalkyl, cycloalkylalkyl, heterocyclyl, and heterocyclylalkyl groups are the same as those described above for alkyl groups. The heterocyclyl groups typically contain 1, 2 or 3 heteroatoms, selected from N, O and S as ring members; and the N or S can be substituted with the groups commonly found on these atoms in heterocyclic systems. As used herein, these terms also include rings that contain a double bond or two, as long as the ring that is attached is not aromatic. The substituted cycloalkyl and heterocyclyl groups also include cycloalkyl or heterocyclic rings fused to an aromatic ring or heteroaromatic ring, provided the point of attachment of the group is to the cycloalkyl or heterocyclyl ring rather than to the aromatic/heteroaromatic ring.

As used herein, "acyl" encompasses groups comprising an alkyl, alkenyl, alkynyl, aryl or arylalkyl radical attached at one of the two available valence positions of a carbonyl carbon atom, and heteroacyl refers to the corresponding groups wherein at least one carbon other than the carbonyl carbon has been replaced by a heteroatom chosen from N, O and S.

Acyl and heteroacyl groups are bonded to any group or molecule to which they are attached through the open valence of the carbonyl carbon atom. Typically, they are C₁-C₈ acyl groups, which include formyl, acetyl, pivaloyl, and benzoyl, and C₂-C₈ heteroacyl groups, which include methoxyacetyl, ethoxycarbonyl, and 4-pyridinoyl.

Similarly, "arylalkyl" and "heteroarylalkyl" refer to aromatic and heteroaromatic ring systems which are bonded to their attachment point through a linking group such as an alkylene, including substituted or unsubstituted, saturated or unsaturated, cyclic or acyclic linkers. Typically the linker is C₁-C₈ alkyl. These linkers may also include a carbonyl group, thus making them able to provide substituents as an acyl or heteroacyl moiety. An aryl or heteroaryl ring in an arylalkyl or heteroarylalkyl group may be substituted with the same substituents described above for aryl groups. Preferably, an arylalkyl group includes a phenyl ring optionally substituted with the groups defined above for aryl groups and a C₁-C₄ alkylene that is unsubstituted or is substituted with one or two C₁-C₄ alkyl groups or heteroalkyl groups, where the alkyl or heteroalkyl groups can optionally cyclize to form a ring such as cyclopropane, dioxolane, or oxacyclopentane. Similarly, a heteroarylalkyl group preferably includes a C₅-C₆ monocyclic heteroaryl group that is optionally substituted with the groups described above as substituents typical on aryl groups and a C₁-C₄ alkylene that is unsubstituted or is substituted with one or two C₁-C₄ alkyl groups or heteroalkyl groups, or it includes an optionally substituted phenyl ring or C₅-C₆ monocyclic heteroaryl and a C₁-C₄ heteroalkylene that is unsubstituted or is substituted with one or two C₁-C₄ alkyl or heteroalkyl groups, where the alkyl or heteroalkyl groups can optionally cyclize to form a ring such as cyclopropane, dioxolane, or oxacyclopentane.

Where an arylalkyl or heteroarylalkyl group is described as optionally substituted, the substituents may be on either the alkyl or heteroalkyl portion or on the aryl or heteroaryl portion of the group. The substituents optionally present on the alkyl or heteroalkyl portion are the same as those described above for alkyl groups generally; the substituents optionally present on the aryl or heteroaryl portion are the same as those described above for aryl groups generally.

"Arylalkyl" groups as used herein are hydrocarbyl groups if they are unsubstituted, and are described by the total number of carbon atoms in the ring and alkylene or similar linker. Thus a benzyl group is a C7-arylalkyl group, and phenylethyl is a C8-arylalkyl.

"Heteroarylalkyl" as described above refers to a moiety comprising an aryl group that is attached through a linking group, and differs from "arylalkyl" in that at least one ring atom of the aryl moiety or one atom in the linking group is a heteroatom selected from N, O and S. The heteroarylalkyl groups are described herein according to the total number of atoms in the ring and linker combined, and they include aryl groups linked through a heteroalkyl linker; heteroaryl groups linked through a hydrocarbyl linker such as an alkylene; and heteroaryl groups linked through a heteroalkyl linker. Thus, for example, C7-heteroarylalkyl would include pyridylmethyl, phenoxy, and N-pyrrolylmethoxy.

"Alkylene" as used herein refers to a divalent hydrocarbyl group; because it is divalent, it can link two other groups together. Typically it refers to -(CH₂)ₙ-where n is 1-8 and preferably n is 1-4, though where specified, an alkylene can also be substituted by other groups, and can be of other lengths, and the open valences need not be at opposite ends of a chain.

In general, any alkyl, alkenyl, alkynyl, acyl, or aryl or arylalkyl group that is contained in a substituent may itself optionally be substituted by additional substituents. The nature of these substituents is similar to those recited with regard to the primary substituents themselves if the substituents are not otherwise described.

"Amino" as used herein refers to -NH₂, but where an amino is described as "substituted" or "optionally substituted", the term includes NR'R" wherein each R' and R" is independently H, or is an alkyl, alkenyl, alkynyl, acyl, aryl, or arylalkyl group, and each of the alkyl, alkenyl, alkynyl, acyl, aryl, or arylalkyl groups is optionally substituted with the substituents described herein as suitable for the corresponding group; the R' and R" groups and the nitrogen atom to which they are attached can optionally form a 3- to 8-membered ring which may be saturated, unsaturated or aromatic and which contains 1-3 heteroatoms independently selected from N, O and S as ring members, and which is optionally substituted with the substituents described as suitable for alkyl groups or, if NR'R" is an aromatic group, it is optionally substituted with the substituents described as typical for heteroaryl groups.

As used herein, the term "carbocycle," "carbocyclyl," or "carbocyclic" refers to a cyclic ring containing only carbon atoms in the ring, whereas the term "heterocycle" or "heterocyclic" refers to a ring comprising a heteroatom. The carbocyclyl can be fully saturated or partially saturated, but non-aromatic. For example, the carbocyclyl encompasses cycloalkyl. The carbocyclic and heterocyclic structures encompass compounds having monocyclic, bicyclic or multiple ring systems; and such systems may mix aromatic, heterocyclic, and carbocyclic rings. Mixed ring systems are described according to the ring that is attached to the rest of the compound being described.

As used herein, the term "heteroatom" refers to any atom that is not carbon or hydrogen, such as nitrogen, oxygen or sulfur. When it is part of the backbone or skeleton of a chain or ring, a heteroatom must be at least divalent, and will typically be selected from N, O, P, and S.

As used herein, the term "alkanoyl" refers to an alkyl group covalently linked to a carbonyl (C=O) group. The term "lower alkanoyl" refers to an alkanoyl group in which the alkyl portion of the alkanoyl group is C₁-C₆. The alkyl portion of the alkanoyl group can be optionally substituted as described above. The term "alkylcarbonyl" can alternatively be used. Similarly, the terms "alkenylcarbonyl" and "alkynylcarbonyl" refer to an alkenyl or alkynyl group, respectively, linked to a carbonyl group.

As used herein, the term "alkoxy" refers to an alkyl group covalently linked to an oxygen atom; the alkyl group can be considered as replacing the hydrogen atom of a hydroxyl group. The term "lower alkoxy" refers to an alkoxy group in which the alkyl portion of the alkoxy group is C₁-C₆. The alkyl portion of the alkoxy group can be optionally substituted as described above. As used herein, the term "haloalkoxy" refers to an alkoxy group in which the alkyl portion is substituted with one or more halo groups.

As used herein, the term "sulfo" refers to a sulfonic acid (-SO₃H) substituent.

As used herein, the term "sulfamoyl" refers to a substituent with the structure -S(O₂)NH₂, wherein the nitrogen of the NH₂ portion of the group can be optionally substituted as described above.

As used herein, the term "carboxyl" refers to a group of the structure - C(O₂)H.

As used herein, the term "carbamyl" refers to a group of the structure - C(O₂)NH₂, wherein the nitrogen of the NH₂ portion of the group can be optionally substituted as described above.

As used herein, the terms "monoalkylaminoalkyl" and "dialkylaminoalkyl" refer to groups of the structure -Alk₁-NH-Alk₂ and -Alk₁-N(Alk₂)(Alk₃), wherein Alk₁, Alk₂, and Alk₃ refer to alkyl groups as described above.

As used herein, the term "alkylsulfonyl" refers to a group of the structure -S(O)₂-Alk wherein Alk refers to an alkyl group as described above. The terms "alkenylsulfonyl" and "alkynylsulfonyl" refer analogously to sulfonyl groups covalently bound to alkenyl and alkynyl groups, respectively. The term "arylsulfonyl" refers to a group of the structure -S(O)₂-Ar wherein Ar refers to an aryl group as described above. The term "aryloxyalkylsulfonyl" refers to a group of the structure -S(O)₂-Alk-O-Ar, where Alk is an alkyl group as described above and Ar is an aryl group as described above. The term "arylalkylsulfonyl" refers to a group of the structure -S(O)₂-AlkAr, where Alk is an alkyl group as described above and Ar is an aryl group as described above.

As used herein, the term "alkyloxycarbonyl" refers to an ester substituent including an alkyl group wherein the carbonyl carbon is the point of attachment to the molecule. An example is ethoxycarbonyl, which is CH₃CH₂OC(O)--. Similarly, the terms "alkenyloxycarbonyl," "alkynyloxycarbonyl," and "cycloalkylcarbonyl" refer to similar ester substituents including an alkenyl group, alkenyl group, or cycloalkyl group respectively. Similarly, the term "aryloxycarbonyl" refers to an ester substituent including an aryl group wherein the carbonyl carbon is the point of attachment to the molecule. Similarly, the term "aryloxyalkylcarbonyl" refers to an ester substituent including an alkyl group wherein the alkyl group is itself substituted by an aryloxy group.

Other combinations of substituents are known in the art and, are described, for example, in United States Patent No. 8,344,162 to Jung et al. For example, the term "thiocarbonyl" and combinations of substituents including "thiocarbonyl" include a carbonyl group in which a double-bonded sulfur replaces the normal double-bonded oxygen in the group. The term "alkylidene" and similar terminology refer to an alkyl group, alkenyl group, alkynyl group, or cycloalkyl group, as specified, that has two hydrogen atoms removed from a single carbon atom so that the group is double-bonded to the remainder of the structure.

The compounds disclosed herein may exist as salts at physiological pH ranges or other ranges. Such salts are described further below. In general, the term "pharmaceutically acceptable salts" is meant to include salts of the active compounds which are prepared with relatively nontoxic acids or bases, depending on the particular substituents found on the compounds described herein. Base addition salts can be obtained by contacting the neutral form of such compounds with a sufficient amount of the desired base, either net or in a suitable inert solvent. Examples of pharmaceutically acceptable base addition salts include sodium, potassium, calcium, ammonium, organic amino, or magnesium salt, or a similar salt. Acid addition salts can be obtained by contacting the neutral form of such compounds with a sufficient amount of the desired acid, either net or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isbutyric, oxalic, maleic, malonic, benzoic, succinic, suberic, fumeric, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like (see, for example, Berge, S. M., et al., "Pharmaceutical Salts", Journal of Pharmaceutical Science, 1977,66, 1-19). Eflornithine contains both basic and acidic functionalities that allow the compounds of the present invention to be converted into either base or acid addition salts.

Accordingly, one aspect of the present invention is a method of treating a glioma comprising the administration of a therapeutically effective quantity of eflornithine or a pharmaceutically acceptable salt thereof to treat glioma by inhibiting progression of DNA mutations caused by chemotherapy agents to reduce the progression or grade of malignancy of the glioma, wherein the glioma is temozolomide recurrent/refractory anaplastic astrocytoma. All pharmaceutically acceptable salt forms, hydrates, and solvates of eflornithine can be used in this method.

Typically, the glioma was previously treated with radiation therapy and adjuvant alkylator therapy and is recurrent/refractory anaplastic glioma. The glioma can have a mutation in one or more genes selected from the group consisting of *IDH1, IDH2, TP53, PTEN,* and *ATRX.* The glioma can have the promoter for *MGMT* methylated.

The eflornithine or pharmaceutically acceptable salt thereof can be administered alone or together with a therapeutically effective quantity of one or more conventional anti-neoplastic agents used for the treatment of glioma. These agents can include, but are not limited to, alkylating agents, antimetabolites, anti-angiogenic agents, EGFR inhibitors, platinum-containing agents, topoisomerase inhibitors, or other classes of agents. For example, but not by way of limitation, these agents can include lomustine (CCNU), carmustine (BCNU), temozolomide, procarbazine, prednisone, vincristine, PCV (a combination of lomustine, procarbazine, and vincristine), carboplatin, carboplatin plus thymidine, carmustine plus temozolomide, erlotinib, carboplatin plus erlotinib, cloretazine, lomustine plus cloretazine, imatinib, hydroxyurea, hydroxyurea plus imatinib, irinotecan, thalidomide, temozolomide plus thalidomide, rilotumumab, cilengitide, *cis*-retinoic acid, celecoxib, *cis*-retinoic acid plus celecoxib, enzastaurin, sirolimus, erlotinib plus sirolimus, fenretinide, gefitinib, lapatinib, temsirolimus, tipifarnib, vorinostat, diaziquone, methotrexate, melphalan, a combination of vincristine, prednisone, and procarbazine, thioguanine, TPDCV (thioguanine, procarbazine, dibromodulcitol, lomustine, vincristine), a combination of nitrogen mustard, vincristine, and procarbazine, tenoposide, and carboplatin plus tenoposide. Other agents and combinations of agents are known in the art.

According to the invention, the eflornithine or pharmaceutically acceptable salt thereof reduces the rate of mutation of the glioma associated with the administration of the alkylating agent temozolomide.

Eflornithine or a pharmaceutically acceptable salt thereof either alone or together with one or more additional agents as described above, can also be used together with radiotherapy.

Additionally, eflornithine or a pharmaceutically acceptable salt thereof either alone or together with one or more additional agents as described above, can also be used with an inhibitor of polyamine transport or a polyamine analog as described above.

Additionally, eflornithine or a pharmaceutically acceptable salt thereof either alone or together with one or more additional agents as described above, can also be used with an S-adenosylmethionine decarboxylase inhibitor as described above.

Eflornithine or a pharmaceutically acceptable salt thereof can also be administered together with other agents such as, but not limited to: (1) a retinoid; (2) glidobactin, syringolin, and other syrbactin compounds; (3) celecoxib and other cyclooxygenase-2 inhibitors; (4) a verinoid; (5) non-steroidal anti-inflammatory agents such as sulindac; (5) castanospermine and castanospermine esters; (6) aziridinyl putrescine compounds such as 1-(4-aminobutyl)aziridine; (7) an interferon; (8) aryl substituted xylopyranoside derivatives; (9) an agent that reduces blood glutamate levels and enhances brain to blood glutamate efflux; (10) chitosan and chitosan derivatives and analogs; (11) 2,4-disulfonyl phenyl tert-butyl nitrone; (12) 3-(4-amino-1-oxo-1,3-dihydro-isoindol-2-yl)-piperidine-2,6-dione; (13) thalidomide; (14) N-2-pyridinyl-2-pyridinecarbothioamide; (15) cambendazole; and (16) inhibitors of histone demethylase.

In another alternative, the eflornithine or pharmaceutically acceptable salt thereof is administered together with an agent that increases the ability of the eflornithine or pharmaceutically acceptable salt thereof to pass through the blood-brain barrier. Typically, the agent that increases the ability of the eflornithine or pharmaceutically acceptable salt thereof to pass through the blood-brain barrier is an agent selected from the group consisting of:
(a) a chimeric peptide of the structure of Formula (D-III): 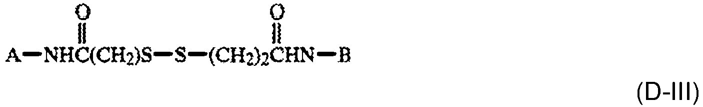 wherein: (A) A is somatostatin, thyrotropin releasing hormone (TRH), vasopressin, alpha interferon, endorphin, muramyl dipeptide or ACTH 4-9 analogue; and (B) B is insulin, IGF-I, IGF-II, transferrin, cationized (basic) albumin or prolactin; or a chimeric peptide of the structure of Formula (D-III) wherein the disulfide conjugating bridge between A and B is replaced with a bridge of Subformula (D-III(a)):

   A-NH(CH₂)₂S-S-B (cleavable linkage) (D-III(a)),

   wherein the bridge is formed using cysteamine and EDAC as the bridge reagents; or a chimeric peptide of the structure of Formula (D-III) wherein the disulfide conjugating bridge between A and B is replaced with a bridge of Subformula (D-III(b)):

   A-NH=CH(CH₂)₃CH=NH-B (non-cleavable linkage) (D-III(b)),

   wherein the bridge is formed using glutaraldehyde as the bridge reagent;
(b) a composition comprising either avidin or an avidin fusion protein bonded to a biotinylated eflornithine or pharmaceutically acceptable salt thereof to form an avidin-biotin-agent complex including therein a protein selected from the group
   consisting of insulin, transferrin, an anti-receptor monoclonal antibody, a cationized protein, and a lectin;
(c) a neutral liposome that is pegylated and incorporates the eflornithine or pharmaceutically acceptable salt thereof, wherein the polyethylene glycol strands are conjugated to at least one transportable peptide or targeting agent;
(d) a humanized murine antibody that binds to the human insulin receptor linked to the eflornithine or pharmaceutically acceptable salt thereof through an avidin-biotin linkage; and
(e) a fusion protein comprising a first segment and a second segment: the first segment comprising a variable region of an antibody that recognizes an antigen on the surface of a cell that after binding to the variable region of the antibody undergoes antibody-receptor-mediated endocytosis, and, optionally, further comprises at least one domain of a constant region of an antibody; and the second segment comprising a protein domain selected from the group consisting of avidin, an avidin mutein, a chemically modified avidin derivative, streptavidin, a streptavidin mutein, and a chemically modified streptavidin derivative, wherein the fusion protein is linked to the eflornithine or pharmaceutically acceptable salt thereof by a covalent link to biotin.

When multiple therapeutic agents are administered, each therapeutic agent can be administered separately, or two or more therapeutic agents can be administered in a single pharmaceutical composition. For example, when three therapeutic agents are to be administered, the following possibilities exist. (1) Each of the three therapeutic agents is administered individually; in this case, each agent can be administered in a separate pharmaceutical composition or as the agent alone without use of a pharmaceutical composition for the agent. Further details on the composition and preparation of pharmaceutical compositions are provided below. In this alternative, zero, one, two, or three separate pharmaceutical compositions can be used. (2) Two of the therapeutic agents are administered together in a single pharmaceutical composition, while the third therapeutic agent is administered separately, either as the agent alone or in a separate pharmaceutical composition. (3) All three therapeutic agents are administered together in a single pharmaceutical composition.

Another aspect of the invention is a pharmaceutical composition for the treatment of glioma comprising:
(1) a therapeutically effective quantity of eflornithine or a pharmaceutically acceptable salt thereof as described above;
(2) optionally, a therapeutically effective quantity of at least one additional agent as described above that can be used together with eflornithine or a pharmaceutically acceptable salt thereof and
(3) a pharmaceutically acceptable carrier.

Conventional pharmaceutically acceptable carriers are known in the art and include, but are not limited to, a sugar, a solvent, a thickener, an emulsifying agent, a diluent, a sweetener, a wetting agent, an organic acid, a coloring agent, a flavoring agent, and a preservative.

In one alternative, a composition according to the present invention can further comprise an agent that increases the ability of the eflornithine or pharmaceutically acceptable salt thereof to pass through the blood-brain barrier. Typically, the agent that increases the ability of the eflornithine or pharmaceutically acceptable salt thereof to pass through the blood-brain barrier is an agent selected from the group consisting of:
(a) a chimeric peptide of the structure of Formula (D-III): 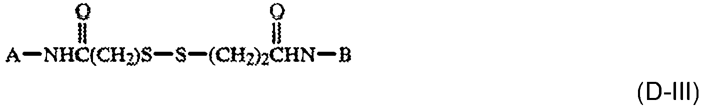 wherein: (A) A is somatostatin, thyrotropin releasing hormone (TRH), vasopressin, alpha interferon, endorphin, muramyl dipeptide or ACTH 4-9 analogue; and (B) B is insulin, IGF-I, IGF-II, transferrin, cationized (basic) albumin or prolactin; or a chimeric peptide of the structure of Formula (D-III) wherein the disulfide conjugating bridge between A and B is replaced with a bridge of Subformula (D-III(a)):

   **A-NH(CH₂)₂S-S-B (cleavable linkage)** (D-III(a)),

   wherein the bridge is formed using cysteamine and EDAC as the bridge reagents; or a chimeric peptide of the structure of Formula (D-III) wherein the disulfide conjugating bridge between A and B is replaced with a bridge of Subformula (D-III(b)):

   **A-NH=CH(CH₂)₃CH=NH-B (non-cleavable linkage)** (D-III(b)),

   wherein the bridge is formed using glutaraldehyde as the bridge reagent;
(b) a composition comprising either avidin or an avidin fusion protein bonded to a biotinylated eflornithine or pharmaceutically acceptable salt thereof to form an avidin-biotin-agent complex including therein a protein selected from the group consisting of insulin, transferrin, an anti-receptor monoclonal antibody, a cationized protein, and a lectin;
(c) a neutral liposome that is pegylated and incorporates the eflornithine or pharmaceutically acceptable salt thereof wherein the polyethylene glycol strands are conjugated to at least one transportable peptide or targeting agent;
(d) a humanized murine antibody that binds to the human insulin receptor linked to the eflornithine or pharmaceutically acceptable salt thereof through an avidin-biotin linkage; and
(e) a fusion protein comprising a first segment and a second segment: the first segment comprising a variable region of an antibody that recognizes an antigen on the surface of a cell that after binding to the variable region of the antibody undergoes antibody-receptor-mediated endocytosis, and, optionally, further comprises at least one domain of a constant region of an antibody; and the second segment comprising a protein domain selected from the group consisting of avidin, an avidin mutein, a chemically modified avidin derivative, streptavidin, a streptavidin mutein, and a chemically modified streptavidin derivative, wherein the fusion protein is linked to the eflornithine or pharmaceutically acceptable salt thereof by a covalent link to biotin.

When the pharmacologically active compound in a pharmaceutical composition according to the present invention possesses a sufficiently acidic, a sufficiently basic, or both a sufficiently acidic and a sufficiently basic functional group, these group or groups can accordingly react with any of a number of inorganic or organic bases, and inorganic and organic acids, to form a pharmaceutically acceptable salt. Exemplary pharmaceutically acceptable salts include those salts prepared by reaction of the pharmacologically active compound with a mineral or organic acid or an inorganic base, such as salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, β-hydroxybutyrates, glycolates, tartrates, methane-sulfonates, propanesulfonates, naphthalene-1-sulfonates, naphthalene-2-sulfonates, and mandelates. If the pharmacologically active compound has one or more basic functional groups, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha-hydroxy acid, such as citric acid or tartaric acid, an amino acid, such as aspartic acid or glutamic acid, an aromatic acid, such as benzoic acid or cinnamic acid, a sulfonic acid, such as *p*-toluenesulfonic acid or ethanesulfonic acid, or the like. If the pharmacologically active compound has one or more acidic functional groups, the desired pharmaceutically acceptable salt may be prepared by any suitable method available in the art, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

In the case of agents that are solids, it is understood by those skilled in the art that the inventive compounds and salts may exist in different crystal or polymorphic forms, all of which are intended to be within the scope of the present invention and specified formulas.

Plasma concentrations in the subjects may be between about 60 µM to about 1000 µM). In some embodiments, the plasma concentration may be between about 200 µM to about 800 µM. In other embodiments, the concentration is about 300 µM to about 600 µM. In still other embodiments the plasma concentration may be between about 400 to about 800 µM. In another alternative, the plasma concentration can be between about 0.5 µM to about 20 µM, typically 1 µM to about 10 µM.

The compositions of the invention may be manufactured using techniques generally known for preparing pharmaceutical compositions, e.g., by conventional techniques such as mixing, dissolving, granulating, dragee-making, levitating, emulsifying, encapsulating, entrapping or lyophilizing. Pharmaceutical compositions may be formulated in a conventional manner using one or more physiologically acceptable carriers, which may be selected from excipients and auxiliaries that facilitate processing of the active compounds into preparations, which can be used pharmaceutically.

Proper formulation is dependent upon the route of administration chosen. For injection, the agents of the invention may be formulated into aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers known in the art. Such carriers enable the compounds of the invention to be formulated as tablets, pills, dragees, capsules, liquids, gels, syrups, slurries, solutions, suspensions and the like, for oral ingestion by a patient to be treated. Pharmaceutical preparations for oral use can be obtained using a solid excipient in admixture with the active ingredient (agent), optionally grinding the resulting mixture, and processing the mixture of granules after adding suitable auxiliaries, if desired, to obtain tablets or dragee cores. Suitable excipients include: fillers such as sugars, including lactose, sucrose, mannitol, or sorbitol; and cellulose preparations, for example, maize starch, wheat starch, rice starch, potato starch, gelatin, gum, methyl cellulose, hydroxypropylmethyl-cellulose, sodium carboxymethylcellulose, or polyvinylpyrrolidone (PVP). If desired, disintegrating agents may be added, such as crosslinked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, polyvinyl pyrrolidone, Carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active agents.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as talc or magnesium stearate, and, optionally, stabilizers. In soft capsules, the active agents may be dissolved or suspended in suitable liquids, such as fatty oils, liquid paraffin, or liquid polyethylene glycols. In addition, stabilizers may be added. All formulations for oral administration should be in dosages suitable for such administration. For buccal administration, the compositions may take the form of tablets or lozenges formulated in conventional manner.

Pharmaceutical formulations for parenteral administration can include aqueous solutions or suspensions. Suitable lipophilic solvents or vehicles include fatty oils such as sesame oil or synthetic fatty acid esters, such as ethyl oleate or triglycerides. Aqueous injection suspensions may contain substances which increase the viscosity of the suspension, such as sodium carboxymethyl cellulose, sorbitol, or dextran. Optionally, the suspension may also contain suitable stabilizers or modulators which increase the solubility or dispersibility of the composition to allow for the preparation of highly concentrated solutions, or can contain suspending or dispersing agents. Pharmaceutical preparations for oral use can be obtained by combining the pharmacologically active agent with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating modulators may be added, such as the cross-linked polyvinyl pyrrolidone, agar, or alginic acid or a salt thereof such as sodium alginate.

Other ingredients such as stabilizers, for example, antioxidants such as sodium citrate, ascorbyl palmitate, propyl gallate, reducing agents, ascorbic acid, vitamin E, sodium bisulfite, butylated hydroxytoluene, BHA, acetylcysteine, monothioglycerol, phenyl-α-naphthylamine, or lecithin can be used. Also, chelators such as EDTA can be used. Other ingredients that are conventional in the area of pharmaceutical compositions and formulations, such as lubricants in tablets or pills, coloring agents, or flavoring agents, can be used. Also, conventional pharmaceutical excipients or carriers can be used. The pharmaceutical excipients can include, but are not necessarily limited to, calcium carbonate, calcium phosphate, various sugars or types of starch, cellulose derivatives, gelatin, vegetable oils, polyethylene glycols and physiologically compatible solvents. Other pharmaceutical excipients are well known in the art. Exemplary pharmaceutically acceptable carriers include, but are not limited to, any and/or all of solvents, including aqueous and non-aqueous solvents, dispersion media, coatings, antibacterial and/or antifungal agents, isotonic and/or absorption delaying agents, and/or the like. The use of such media and/or agents for pharmaceutically active substances is well known in the art. Except insofar as any conventional medium, carrier, or agent is incompatible with the active ingredient or ingredients, its use in a composition according to the present invention is contemplated. Supplementary active ingredients can also be incorporated into the compositions, particularly as described above. For administration of any of the compounds used in the present invention, preparations should meet sterility, pyrogenicity, general safety, and purity standards as required by the FDA Office of Biologics Standards or by other regulatory organizations regulating drugs.

For administration intranasally or by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of gelatin for use in an inhaler or insufflator and the like may be formulated containing a powder mix of the compound and a suitable powder base such as lactose or starch.

The compounds may be formulated for parenteral administration by injection, e.g., by bolus injection or continuous infusion. Formulations for injection may be presented in unit-dosage form, e.g., in ampules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active agents may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents, which increase the solubility of the compounds to allow for the preparation of highly concentrated solutions.

Alternatively, the active ingredient may be in powder form for constitution with a suitable vehicle, e.g., sterile pyrogen-free water, before use. The compounds may also be formulated in rectal compositions such as suppositories or retention enemas, e.g., containing conventional suppository bases such as cocoa butter or other glycerides.

In addition to the formulations described above, the compounds may also be formulated as a depot preparation. Such long-acting formulations may be administered by implantation (for example, subcutaneously or intramuscularly) or by intramuscular injection. Thus, for example, the compounds may be formulated with suitable polymeric or hydrophobic materials (for example, as an emulsion in an acceptable oil) or ion-exchange resins, or as sparingly soluble derivatives, for example, as a sparingly soluble salt.

The pharmaceutical compositions also may comprise suitable solid- or gel-phase carriers or excipients. Examples of such carriers or excipients include calcium carbonate, calcium phosphate, sugars, starches, cellulose derivatives, gelatin, and polymers such as polyethylene glycols.

A pharmaceutical composition can be administered by a variety of methods known in the art. The routes and/or modes of administration vary depending upon the desired results. Depending on the route of administration, the pharmacologically active agent may be coated in a material to protect the therapeutic agent or agents from the action of acids and other compounds that may inactivate the agent. Conventional pharmaceutical practice can be employed to provide suitable formulations or compositions for the administration of such pharmaceutical compositions to subjects. Any appropriate route of administration can be employed, for example, but not limited to, intravenous, parenteral, intraperitoneal, intravenous, transcutaneous, subcutaneous, intramuscular, or oral administration. Depending on the severity of the malignancy or other disease, disorder, or condition to be treated, as well as other conditions affecting the subject to be treated, either systemic or localized delivery of the pharmaceutical composition can be used in the course of treatment. The pharmaceutical composition as described above can be administered together with additional therapeutic agents intended to treat a particular disease or condition, which may be the same disease or condition that the pharmaceutical composition is intended to treat, which may be a related disease or condition, or which even may be an unrelated disease or condition.

The aspects disclosed in the following paragraph are not encompassed by the present invention.

As detailed above, eflornithine or pharmaceutically acceptable salts thereof have been described as effective for the treatment of glioma, in particular with respect to inhibiting or slowing the progression of glioma to a higher grade. However, all forms of cancer are associated with mutation in malignant cells, so eflornithine or derivatives or analogs thereof can be similarly administered to inhibit or slow the advance of other malignancies as well by preventing mutation in the malignant cells. Although eflornithine or its derivatives or analogs can be used to slow the advance of and prevent mutation in many types of cancers, in particular, eflornithine or its derivatives or analogs can be used to treat neuroblastoma. Eflornithine or its derivatives or analogs increase the concentration of p21 (waf1/cip1) and p27kip-1 and this acts as a cause of cell cycle arrest. Among the tumor types for which such observations have been made are leukemia, pancreatic cancer, neuroblastoma, mammary tumors, and gastric cancer. This is addressed in the following references: (i) P.M. Bauer et al., "Role of p42/p44 Mitogen-Activated-Protein Kinase and p21waf1/cip1 in the Regulation of Vascular Smooth Muscle Cell Proliferation by Nitric Oxide," Proc. Natl. Acad. Sci. USA 98: 12802-12807 (2001); (ii) S.H. Choi et al., "Polyamine-Depletion Induces p27Kip1 and Enhances Dexamethasone-Induced G1 Arrest and Apoptosis in Human T lymphoblastic Leukemia Cells," Leuk. Res. 24: 119-127 (2000); (iii) H. Guo et al., "RhoA Stimulates IEC-6 Cell Proliferation by Increasing Polyamine-Dependent Cdk2 Activity," Am. J. Physiol. Gastrointest. Liver Physiol. 285: G704-713 (2003); (iv) L. Li et al., "JunD Stabilization Results in Inhibition of Normal Intestinal Epithelial Cell Growth through P21 after Polyamine Depletion," Gastroenterology 123: 764-779 (2002); (v) M. Li et al., "Chemoprevention of Mammary Carcinogenesis in a Transgenic Mouse Model by Alpha-Difluoromethylornithine (DFMO) in the Diet Is associated with Decreased Cyclin D1 Activity," Oncogene 22: 2568-2572 (2003); (vi) A. Mohammed et al., "Eflornithine (DFMO) Prevents Progression of Pancreatic Cancer by Modulating Ornithine Decarboxylase Signaling," Cancer Prev. Res. 7: 1198-1209 (2014); (vii) T. Nemoto et al., "p53 Independent G(1) arrest Induced by DL-Alpha-Difluoromethylornithine," Biochem. Biophys. Res. Commun. 280: 848-854 (2001); (viii) "R.M. Ray et al., "Polyamine Depletion Arrests Cell Cycle and Induces Inhibitors p21(Waf1/Cip1), p27(Kip1), and p53 in IEC-6 Cells," Am. J Physiol. 276: C684-691 (1999); (ix) R.J. Rounbehler et al., "Targeting Ornithine Decarboxylase Impairs Development of MYCN-Amplified Neuroblastoma," Cancer Res. 69: 547-553 (2009); (x) J. Singh et al., "Modulation of Azoxymethane-Induced Mutational Activation of ras Protooncogenes by Chemopreventive Agents in Colon Carcinogenesis," Carcinogenesis 15: 1317-1323 (1994); (xi) R. Singh et al., "Activation of Caspase-3 Activity and Apoptosis in MDA-MB-468 Cells by N(omega)-Hydroxy-L-Arginine, an Inhibitor of Arginase, Is Not Solely Dependent on Reduction in Intracellular Polyamines," Carcinogenesis 22: 1863-1869 (2001); (xii) L. Tao et al., "Altered Expression of c-myc, p16 and p27 in Rat Colon Tumors and Its Reversal by Short-Term Treatment with Chemopreventive Agents." Carcinogenesis 23: 1447-1454 (2002); (xiii) C.J. Wallick et al., "Key Role for p27Kip1, Retinoblastoma Protein Rb, and MYCN in Polyamine Inhibitor-Induced G1 Cell Cycle Arrest in MYCN-Amplified Human Neuroblastoma Cells," Oncogene 24: 5606-5618 (2005); (xiv) Q. Xiang et al., "[Apoptotic Induction of Human Lung Carcinoma A549 Cells by DFMO through Fas/FasL Pathway]," Ai Zheng 12: 1260-1263 (2003); and References (9) and (10), below

The invention is illustrated by the following Example.

### EXAMPLE

### Toxicity of Eflornithine

The toxicity produced by eflornithine from the original Phase 2 randomized study (Levin et al., 1992) of AG and GBM patients treated at recurrence is shown in Table 1. In this study, eflornithine was administered at a dose of 3.6 g/m² every 8 hours for 14-days out of 21-days. At this dose, 13%, 16%, 3%, and 1% of patients receiving eflornithine reported diarrhea at toxicity grades 1 through 4, respectively. In exceptional cases, it was noted that dividing the daily eflornithine dosage from 3 times daily to 4 to 6 smaller doses proved an effective relief from the diarrhea which was considered to be due to the osmotic load of the oral eflornithine on the gastrointestinal tract. In this study, hematological toxicity appeared to be very acceptable requiring little in the way of eflornithine dose reduction.

**Table 1**

| Summary of Relevant Adverse Events Attributed to Eflornithine in a Phase 2 Study on Tumor Recurrence/Progression (N=89) | | | | | | | | |
|---|---|---|---|---|---|---|---|---|
| **Adverse Events** | **Toxicity Grade 1** | | **Toxicity Grade 2** | | **Toxicity Grade 3** | | **Toxicity Grade 4** | |
| | **n** | **(%)** | **N** | **(%)** | **n** | **(%)** | **n** | **(%)** |
| Anemia | 1 | (1.1) | 8 | (9.0) | - | - | - | - |
| Leukopenia* | 16 | (18.0) | 14 | (15.7) | 2 | (2.2) | - | - |
| Granulocytopenia** | 6 | (6.7) | 8 | (9.0) | 5 | (5.6) | - | - |
| Thrombocytopenia | 1 | (1.1) | 2 | (2.2) | 4 | (4.5) | - | - |
| Hearing Loss | 4 | (4.5) | 6 | (6.7) | 9 | (10.1) | 2 | (2.2) |
| Ototoxicity | 7 | (7.9) | 5 | (5.6) | 6 | (6.7) | - | - |
| Anorexia | 1 | (1.1) | 1 | (1.1) | - | - | - | - |
| Diarrhea | 11 | (12.4) | 15 | (16.9) | 4 | (4.5) | 1 | (1.1) |
| Nausea/Vomiting | 8 | (9.0) | 4 | (4.5) | 3 | (3.4) | 1 | (1.1) |
| Fatigue | 3 | (3.4) | 5 | (5.6) | - | - | - | - |
| Malaise | 1 | (1.1) | 1 | (1.1) | - | - | - | - |
| Headache | - | - | 1 | (1.1) | - | - | - | - |

| | | | | | | | | |
|---|---|---|---|---|---|---|---|---|
| * Leukopenia reflects primarily reduction in neutrophils & lymphocytes ** Granulocytopenia = neutropenia | | | | | | | | |

The toxicity produced by eflornithine in combination with PCV in a Phase 3 study (7) of eflornithine with PCV versus PCV for the adjuvant, post-irradiation, chemotherapy of newly diagnosed AG and GBM patients is summarized in Table 2 (N=500). As used herein, the term "toxicity" refers to any one of the side effects referred to above in Table 2, regardless of the severity of the side effect or its possible effect on the course of treatment. In this table, the differential toxicities that may be attributable to eflornithine, 3.0 g/m² every 8 hours for 14 days out of 28 days, are summarized. The only adverse event that was statistically significantly elevated in the eflornithine with PCV arm versus the PCV only arm was diarrhea (p = 0.013)

**Table 2**

| Summary of Relevant Adverse Events Attributable to Eflornithine in a Phase 3 AG and GBM Study in Eflornithine-PCV Treatment vs PCV Treatment (N=500) | | | | | | | | | | | | |
|---|---|---|---|---|---|---|---|---|---|---|---|---|
| **Adverse Events (%)** | **Toxicity Grade 1** | | | **Toxicity Grade 2** | | | **Toxicity Grade 3** | | | **Toxicity Grade 4** | | |
| | **Eflornithi ne-PCV** | **PCV** | **Excess AEs due to Eflornithine** | **Eflornithine-PCV** | **PCV** | **Excess AEs due to Eflornith ine** | **Eflornithi ne-PCV** | **PC V** | **Excess AEs due to Eflornithine** | **Eflornithi ne**-**PCV** | **PC V** | **Excess AEs due to Eflornithine** |
| Anemia | 36.7 | 24. 2 | 12.5 | 31.9 | 16. 3 | 15.6 | 7.7 | 3.2 | 4.5 | 2.0 | 0. 0 | 2.0 |
| Leukopenia* | 58.1 | 61. 5 | -3.4 | 53.6 | 52. 4 | 1.2 | 27.8 | 27. 8 | 0.0 | 4.0 | 3. 2 | 0.9 |
| Granulocytope nia** | 52.0 | 49. 6 | 2.4 | 43.5 | 42. 5 | 1.1 | 26.6 | 30. 6 | -3.9 | 11.3 | 9. 5 | 1.8 |
| Thrombocytop enia | 41.1 | 36. 5 | 4.6 | 39.5 | 29. 0 | 10.5 | 20.2 | 16. 7 | 3.5 | 5.2 | 3. 2 | 2.1 |
| Hearnig loss | 2.4 | 2.4 | 0.0 | 2.8 | 1.6 | 1.2 | 0.8 | 0.0 | 0.8 | 0.0 | 0. 0 | 0.0 |
| Anorexia | 7.7 | 9.1 | -1.5 | 6.0 | 3.6 | 2.5 | 1.2 | 0.0 | 1.2 | 0.0 | 0. 0 | 0.0 |
| Diarrhea | 28.2 | 2.8 | 25.4 | 19.0 | 1.6 | 17.4 | 6.0 | 0.0 | 6.0 | 0.8 | 0. 0 | 0.8 |
| Nausea only | 36.7 | 35. 7 | 1.0 | 23.0 | 23. 0 | 0.0 | 7.7 | 6.7 | 0.9 | 0.0 | 0. 0 | 0.0 |
| Vomiting | 24.6 | 19. 4 | 5.2 | 21.0 | 19. 8 | 1.1 | 7.7 | 3.2 | 4.5 | 0.4 | 1. 6 | -1.2 |
| Constipation | 4.0 | 6.0 | -1.9 | 4.8 | 5.6 | -0.7 | 0.4 | 0.0 | 0.4 | 0.4 | 0. 0 | 0.4 |
| Fatigue | 18.5 | 13. 9 | 4.7 | 12.5 | 5.6 | 6.9 | 1.6 | 0.8 | 0.8 | 0.0 | 0. 0 | 0.0 |
| Headache | 3.6 | 5.6 | -1.9 | 4.0 | 2.8 | 1.3 | 1.2 | 0.8 | 0.4 | 0.0 | 0. 0 | 0.0 |
| Pruritis and itching | 4.4 | 5.2 | -0.7 | 8.1 | 7.1 | 0.9 | 4.0 | 4.4 | -0.3 | 0.0 | 0. 0 | 0.0 |

| | | | | | | | | | | | | |
|---|---|---|---|---|---|---|---|---|---|---|---|---|
| ^{†} p = 0.013 * Leukopenia reflects primarily reduction in neutrophils & lymphocytes ** Granulocytopenia = neutropenia | | | | | | | | | | | | |

These publications are referred to herein by the numbers provided below. The inclusion of any publication in this list of publications is not to be taken as an admission that any publication referred to herein is prior art.
**1.** Metcalf R, Bey P, Danzin C, Jung MJ, Casara P, Vevert JP. Catalytic irreversible inhibition of mammalian ornithine decarboxylase (EC 4.1.1.17) by substrate and analog product analogs. J Am Chem Soc. 1978;100:2551-2552.
**2.** Bacchi CJ, Garofalo J, Mockenhaupt D, et al. In vivo effects of alpha-DL-difluoromethylornithine on the metabolism and morphology of Trypanosoma brucei brucei. Mol Biochem Parasitol. Mar 1983;7(3):209-225.
**3.** Bacchi CJ, Nathan HC, Hutner SH, McCann PP, Sjoerdsma A. Polyamine metabolism: a potential therapeutic target in trypanosomes. Science. Oct 17 1980;210(4467):332-334.
**4.** Shantz LM, Levin VA. Regulation of ornithine decarboxylase during oncogenic transformation: mechanisms and therapeutic potential. Amino Acids. Aug 2007;33(2):213-223.
**5.** Childs AC, Mehta DJ, Gerner EW. Polyamine-dependent gene expression. Cell Mol Life Sci. Jul 2003;60(7):1394-1406.
**6.** Gerner EW, Meyskens FL, Jr. Polyamines and cancer: old molecules, new understanding. Nat Rev Cancer. Oct 2004;4(10):781-792.
**7.** Levin VA, Hess KR, Choucair A, et al. Phase III randomized study of postradiotherapy chemotherapy with combination alpha-difluoromethylornithine-PCV versus PCV for anaplastic gliomas. Clinical Cancer Research. Mar 2003;9(3):981-990.
**>Final** Levin VA, Hess KR, Choucair AK, et al. Final report for evaluable patients treated on DM92-035, phase III randomized study of post-irradiation PCV versus DFMO-PCV, for anaplastic gliomas (AG). Neuro Oncol. 2012;14(Supplement 6):vi74**.**
**9.** Koomoa DL, Yco LP, Borsics T, Wallick CJ, Bachmann AS. Ornithine decarboxylase inhibition by DFMO activates opposing signaling pathways via phosphorylation of both Akt/PKB and p27Kip1 in neuroblastoma. Cancer Res. Dec 1 2008;68(23):9825-9831.
**10.** Koomoa DL, Geerts D, Lange I, et al. DFMO/eflornithine inhibits migration and invasion downstream of MYCN and involves p27Kip1 activity in neuroblastoma. Int J Oncol. Apr 2013;42(4):1219-1228.
**11.** Johnson BE, Mazor T, Hong C, et al. Mutational Analysis Reveals the Origin and Therapy-Driven Evolution of Recurrent Glioma. Science. Dec 12 2013.
**12.** Hunter C, Smith R, Cahill DP, et al. A hypermutation phenotype and somatic MSH6 mutations in recurrent human malignant gliomas after alkylator chemotherapy. Cancer Res. Apr 15 2006;66(8):3987-3991.
**13.** Yip S, Miao J, Cahill DP, et al. MSH6 mutations arise in glioblastomas during temozolomide therapy and mediate temozolomide resistance. Clin Cancer Res. Jul 15 2009;15(14):4622-4629.
**14.** The Cancer Genome Atlas Research Network. Comprehensive genomic characterization defines human glioblastoma genes and core pathways. Nature. 2008;455:1061-1068.
**15.** Bodell WJ, Gaikwad NW, Miller D, Berger MS. Formation of DNA adducts and induction of lacl mutations in Big Blue Rat-2 cells treated with temozolomide: implications for the treatment of low-grade adult and pediatric brain tumors. Cancer Epidemiol Biomarkers Prev. Jun 2003;12(6):545-551.
**16.** Einspahr JG, Nelson MA, Saboda K, Warneke J, Bowden GT, Alberts DS. Modulation of biologic endpoints by topical difluoromethylornithine (DFMO), in subjects at high-risk for nonmelanoma skin cancer. Clin Cancer Res. Jan 2002;8(1):149-155.
**17.** Hoshino T, Prados M, Wilson CB, Cho KG, Lee KS, Davis RL. Prognostic implications of the bromodeoxyuridine labeling index of human gliomas. J Neurosurg. 1989;71(3):335-341.
**18.** Labrousse F, Daumas-Duport C, Batorski L, Hoshino T. Histological grading and bromodeoxyuridine labeling index of astrocytomas. Comparative study in a series of 60 cases. J Neurosurg. 1991;75(2):202-205.
**19.** Prados MD, Krouwer HG, Edwards MS, Cogen PH, Davis RL, Hoshino T. PROLIFERATIVE POTENTIAL AND OUTCOME IN PEDIATRIC ASTROCYTIC TUMORS. J Neurooncol. 1992;13(3):277-282.
**20.** Hoshino T, Ahn D, Prados MD, Lamborn K, Wilson CB. Prognostic significance of the proliferative potential of intracranial gliomas measured by bromodeoxyuridine labeling. Int J Cancer. 1993 1993;53(4):550-555.
**21.** Ito S, Chandler KL, Prados MD, et al. Proliferative potential and prognostic evaluation of low-grade astrocytomas. J Neuro-Oncol. 1994 1994;19(1):1-9.
**22.** Onda K, Davis RL, Shibuya M, Wilson CB, Hoshino T. Correlation between the bromodeoxyuridine labeling index and the MIB-1 and Ki-67 proliferating cell indices in cerebral gliomas. Cancer. 1994 1994;74(7):1921-1926.
**23.** Kajiwara Y, Panchabhai S, Levin VA. A new preclinical 3-dimensional agarose colony formation assay. Technol Cancer Res Treat. Aug 2008;7(4):329-334.
**24.** Kajiwara Y, Panchabhai S, Liu DD, Kong M, Lee JJ, Levin VA. Melding a New 3-Dimensional Agarose Colony Assay with the E(max) Model to Determine the Effects of Drug Combinations on Cancer Cells. Technol Cancer Res Treat. Apr 2009;8(2):163-176.
**25.** Levin VA, Panchabhai SC, Shen L, Kornblau SM, Qiu Y, Baggerly KA. Different changes in protein and phosphoprotein levels result from serum starvation of high-grade glioma and adenocarcinoma cell lines. J Proteome Res. Jan 2010;9(1):179-191.
**26.** Levin VA, Panchabhai S, Shen L, Baggerly KA. Protein and phosphoprotein levels in glioma and adenocarcinoma cell lines grown in normoxia and hypoxia in monolayer and three-dimensional cultures. Proteome Sci. Jan 25 2012;10(1):5.

### ADVANTAGES OF THE INVENTION

The present invention, including the methods and compositions described herein, provides a novel and effective method for the treatment of glioma, wherein the glioma is temozolomide recurrent/refractory anaplastic astrocytoma. Methods and compositions of the present invention can protect against progression of anaplastic gliomas (especially anaplastic astrocytoma) to a more malignant phenotype, such as glioblastoma. These methods and compositions are well-tolerated, do not produce significant side effects, and can be used together with other anti-neoplastic agents.

Methods according to the present invention possess industrial applicability for the preparation of a medicament for the treatment of glioma, wherein the glioma is temozolomide recurrent/refractory anaplastic astrocytoma. Compositions according to the present invention possess industrial applicability as pharmaceutical compositions, particularly for the treatment of glioma, wherein the glioma is temozolomide recurrent/refractory anaplastic astrocytoma.

The method claims of the present invention provide specific method steps that are more than general applications of laws of nature and require that those practicing the method steps employ steps other than those conventionally known in the art, in addition to the specific applications of laws of nature recited or implied in the claims, and thus confine the scope of the claims to the specific applications recited therein. In some contexts, these claims are directed to new ways of using an existing drug.

The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed. Thus, it should be understood that although the present invention has been specifically disclosed by preferred embodiments and optional features, modification and variation of the inventions herein disclosed can be resorted by those skilled in the art, and that such modifications and variations are considered to be within the scope of the inventions disclosed herein. The inventions have been described broadly and generically herein. Each of the narrower species and subgeneric groupings falling within the scope of the generic disclosure also form part of these inventions. This includes the generic description of each invention with a proviso or negative limitation removing any subject matter from the genus, regardless of whether or not the excised materials specifically resided therein.

In addition, where features or aspects of an invention are described in terms of the Markush group, those schooled in the art will recognize that the invention is also thereby described in terms of any individual member or subgroup of members of the Markush group. It is also to be understood that the above description is intended to be illustrative and not restrictive. Many embodiments will be apparent to those of in the art upon reviewing the above description. The scope of the invention should therefore, be determined not with reference to the above description, but should instead be determined with reference to the appended claims, along with the full scope of equivalents to which such claims are entitled.

## Claims

1. Eflornithine or a pharmaceutically acceptable salt thereof for use in a method for the treatment of temozolomide recurrent/refractory anaplastic astrocytoma in a subject.

2. Eflornithine or a pharmaceutically acceptable salt thereof for use according to claim 1, wherein the eflornithine or pharmaceutically acceptable salt thereof is administered together with an anti-neoplastic agent.

3. Eflornithine or a pharmaceutically acceptable salt thereof for use according to claim 2, wherein the anti-neoplastic agent is lomustine.

4. Eflornithine or a pharmaceutically acceptable salt thereof for use according to claim 1, wherein the eflornithine or pharmaceutically acceptable salt thereof is a racemic mixture of D-eflornithine and L-eflornithine.

5. Eflornithine or a pharmaceutically acceptable salt thereof for use according to claim 1, wherein the eflornithine or pharmaceutically acceptable salt thereof is D-eflornithine.

6. Eflornithine or a pharmaceutically acceptable salt thereof for use according to claim 1, wherein the eflornithine or pharmaceutically acceptable salt thereof is L-eflornithine.

7. Eflornithine or a pharmaceutically acceptable salt thereof for use according to claim 1, wherein the eflornithine or pharmaceutically acceptable salt thereof is administered to the subject for two weeks, every three weeks.

8. Eflornithine or a pharmaceutically acceptable salt thereof for use according to claim 7, further comprising continuing the method for years.

9. Eflornithine or a pharmaceutically acceptable salt thereof for use according to claim 1, wherein the eflornithine or pharmaceutically acceptable salt thereof is a pharmaceutically acceptable salt.

10. Eflornithine or a pharmaceutically acceptable salt thereof for use according to claim 9, wherein the pharmaceutically acceptable salt is a hydrochloric acid salt.

11. Eflornithine or a pharmaceutically acceptable salt thereof for use according to claim 1, wherein the eflornithine or pharmaceutically acceptable salt thereof is administered orally.

12. Eflornithine or a pharmaceutically acceptable salt thereof for use according to claim 11, wherein the eflornithine or pharmaceutically acceptable salt thereof is administered orally as an aqueous solution.

13. Eflornithine or a pharmaceutically acceptable salt thereof for use according to claim 1, wherein the eflornithine or pharmaceutically acceptable salt thereof is administered together with or adjuvant to radiotherapy.

14. Eflornithine or a pharmaceutically acceptable salt thereof for use according to claim 1, wherein the subject has driver mutations in the RB and AKT-mTOR pathways.

15. Eflornithine or a pharmaceutically acceptable salt thereof for use according to claim 1, wherein the subject has a mutation in one or more genes selected from the group consisting of IDH1, IDH2, TP53, PTEN, and ATRX.

16. Eflornithine or a pharmaceutically acceptable salt thereof for use according to claim 15, wherein the mutation is in the IDH1 gene.

## Patentansprüche

1. Eflornithin oder pharmazeutisch unbedenkliches Salz davon zur Verwendung bei einem Verfahren zur Behandlung von mit Temozolomid rezidivem/refraktärem anaplastischem Astrozytom bei einem Individuum.

2. Eflornithin oder pharmazeutisch unbedenkliches Salz davon zur Verwendung nach Anspruch 1, wobei das Eflornithin oder pharmazeutisch unbedenkliche Salz davon zusammen mit einem antineoplastischen Mittel verabreicht wird.

3. Eflornithin oder pharmazeutisch unbedenkliches Salz davon zur Verwendung nach Anspruch 2, wobei es sich bei dem antineoplastischen Mittel um Lomustin handelt.

4. Eflornithin oder pharmazeutisch unbedenkliches Salz davon zur Verwendung nach Anspruch 1, wobei es sich bei dem Eflornithin oder pharmazeutisch unbedenklichen Salz davon um ein racemisches Gemisch von D-Eflornithin und L-Eflornithin handelt.

5. Eflornithin oder pharmazeutisch unbedenkliches Salz davon zur Verwendung nach Anspruch 1, wobei es sich bei dem Eflornithin oder pharmazeutisch unbedenklichen Salz davon um D-Eflornithin handelt.

6. Eflornithin oder pharmazeutisch unbedenkliches Salz davon zur Verwendung nach Anspruch 1, wobei es sich bei dem Eflornithin oder pharmazeutisch unbedenklichen Salz davon um L-Eflornithin handelt.

7. Eflornithin oder pharmazeutisch unbedenkliches Salz davon zur Verwendung nach Anspruch 1, wobei das Eflornithin oder pharmazeutisch unbedenkliche Salz davon dem Individuum alle drei Wochen jeweils zwei Wochen verabreicht wird.

8. Eflomithin oder pharmazeutisch unbedenkliches Salz davon zur Verwendung nach Anspruch 7, ferner umfassend jahrelanges Fortsetzen des Verfahrens.

9. Eflornithin oder pharmazeutisch unbedenkliches Salz davon zur Verwendung nach Anspruch 1, wobei es sich bei dem Eflornithin oder pharmazeutisch unbedenklichen Salz davon um ein pharmazeutisch unbedenkliches Salz handelt.

10. Eflornithin oder pharmazeutisch unbedenkliches Salz davon zur Verwendung nach Anspruch 9, wobei es sich bei dem pharmazeutisch unbedenklichen Salz um ein Salzsäuresalz handelt.

11. Eflornithin oder pharmazeutisch unbedenkliches Salz davon zur Verwendung nach Anspruch 1, wobei das Eflornithin oder pharmazeutisch unbedenkliche Salz davon oral verabreicht wird.

12. Eflornithin oder pharmazeutisch unbedenkliches Salz davon zur Verwendung nach Anspruch 11, wobei das Eflornithin oder pharmazeutisch unbedenkliche Salz davon oral als wässrige Lösung verabreicht wird.

13. Eflornithin oder pharmazeutisch unbedenkliches Salz davon zur Verwendung nach Anspruch 1, wobei das Eflornithin oder pharmazeutisch unbedenkliche Salz davon zusammen mit oder als Adjuvans bei Radiotherapie verabreicht wird.

14. Eflornithin oder pharmazeutisch unbedenkliches Salz davon zur Verwendung nach Anspruch 1, wobei das Individuum Treibermutationen in den RB- und AKT-mTOR-Wegen aufweist.

15. Eflornithin oder pharmazeutisch unbedenkliches Salz davon zur Verwendung nach Anspruch 1, wobei das Individuum eine Mutation in einem oder mehreren Genen ausgewählt aus der Gruppe bestehend aus IDH1, IDH2, TP53, PTEN und ATRX aufweist.

16. Eflornithin oder pharmazeutisch unbedenkliches Salz davon zur Verwendung nach Anspruch 15, wobei sich die Mutation im IDH1-Gen befindet.

## Revendications

1. Éflornithine ou sel de celle-ci acceptable d'un point de vue pharmaceutique destiné(e) à être utilisé(e) dans un procédé destiné au traitement d'un astrocytome anaplasique récurrent/réfractaire au témozolomide chez un sujet.

2. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant administré(e) conjointement avec un agent antinéoplasique.

3. Éflornithine ou sel de celle-ci acceptable d'un point de vue pharmaceutique destiné(e) à être utilisé(e) selon la revendication 2, dans laquelle/lequel l'agent antinéoplasique est la lomustine.

4. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant un mélange racémique de D-éflornithine et de L-éflornithine.

5. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant la D-éflornithine.

6. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant la L-éflornithine.

7. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant administré(e) au sujet pendant deux semaines, toutes les trois semaines.

8. Éflornithine ou sel de celle-ci acceptable d'un point de vue pharmaceutique destiné(e) à être utilisé(e) selon la revendication 7, comprenant en outre la continuation du procédé pendant des années.

9. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant un sel acceptable d'un point de vue pharmaceutique.

10. Éflornithine ou sel de celle-ci acceptable d'un point de vue pharmaceutique destiné(e) à être utilisé(e) selon la revendication 9, le sel acceptable d'un point de vue pharmaceutique étant un sel de l'acide chlorhydrique.

11. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant administré(e) oralement.

12. Éflornithine ou sel de celle-ci acceptable d'un point de vue pharmaceutique destiné(e) à être utilisé(e) selon la revendication 11, l'éflornithine ou le sel de celle-ci acceptable d'un point de vue pharmaceutique étant administré(e) oralement sous forme de solution aqueuse.

13. Éflornithine ou sel de celle-ci acceptable d'un point de vue pharmaceutique destiné(e) à être utilisé(e) selon la revendication 1, l'éflornithine ou le sel de celle-ci acceptable d'un point de vue pharmaceutique étant administré(e) conjointement avec une radiothérapie ou comme adjuvant à celle-ci.

14. Éflornithine ou sel de celle-ci acceptable d'un point de vue pharmaceutique destiné(e) à être utilisé(e) selon la revendication 1, dans laquelle/lequel le sujet a des mutations pilotes dans les voies de RB et AKT-mTOR.

15. Éflornithine ou sel de celle-ci acceptable d'un point de vue pharmaceutique destiné(e) à être utilisé(e) selon la revendication 1, dans laquelle/lequel le sujet a une mutation dans un ou plusieurs gène(s) choisi(s) dans le groupe constitué par les IDH1, IDH2, TP53, PTEN et ATRX.

16. Éflornithine ou sel de celle-ci acceptable d'un point de vue pharmaceutique destiné(e) à être utilisé(e) selon la revendication 15, dans laquelle/lequel la mutation est dans le gène IDH1.
